(19) Europäisches Patentamt
European Patent Office
Office européen des brevets

(11) EP 0 716 082 B1

(12) **EUROPÄISCHE PATENTSCHRIFT**

(45) Veröffentlichungstag und Bekanntmachung des Hinweises auf die Patenterteilung:
**07.03.2001 Patentblatt 2001/10**

(21) Anmeldenummer: **95118609.7**

(22) Anmeldetag: **27.11.1995**

(51) Int Cl.[7]: **C07D 215/14**, C07D 401/12, A61K 31/47, C07D 215/26, C07D 217/16, C07D 241/42, C07D 333/56, C07D 215/18, C07C 235/34, C07D 405/12

(54) **Bicyclisch substituierte Oxy-phenyl-(phenyl) glycinolamide mit antiatherosklerotischer Wirkung**

Oxy-phenyl-(phenyl)glycinolamides substituted with a bi-cycle having antiatherosclerotic activity

Oxy-phényl-(phényl)glycinolamides substitués avec un bicycle ayant une activité antiathérosclérotique

(84) Benannte Vertragsstaaten:
**CH DE FR GB IT LI**

(30) Priorität: **09.12.1994 DE 4443891**

(43) Veröffentlichungstag der Anmeldung:
**12.06.1996 Patentblatt 1996/24**

(73) Patentinhaber: **BAYER AG**
**51368 Leverkusen (DE)**

(72) Erfinder:
- **Müller, Ulrich, Dr.**
  **D-42111 Wuppertal (DE)**
- **Connell, Richard, Dr.**
  **Trumbull, CT 06611 (US)**
- **Goldmann, Siegfried, Dr.**
  **D-42327 Wuppertal (DE)**
- **Mohrs, Klaus-Helmut, Dr.**
  **D-42113 Wuppertal (DE)**
- **Raddatz, Siegfried Dr.**
  **D-51065 Köln (DE)**
- **Matzke, Michael Dr.**
  **D-42113 Wuppertal (DE)**
- **Grützmann, Rudi**
  **D- 42657 Solingen (DE)**
- **Beuck, Martin Dr.**
  **Nilford, CT 06460 (US)**
- **Wohlfeil, Stefan Dr.**
  **D-40724 Hilden (DE)**
- **Bischoff, Hilmar Dr.**
  **D-42113 Wuppertal (DE)**
- **Denzer, Dirk Dr.**
  **D-42115 Wuppertal (DE)**

(56) Entgegenhaltungen:
**EP-A- 0 293 880**
**EP-A- 0 344 519**
**EP-A- 0 574 774**
**EP-A- 0 610 698**
**EP-A- 0 667 342**
**WO-A-92/09561**
**WO-A-92/10468**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 716 082 B1

Printed by Jouve, 75001 PARIS (FR)

## Beschreibung

Die vorliegende Erfindung betrifft bicyclisch substituierte Oxy-phenyl-(phenyl)glycinolamide, insbesondere ihre Verwendung zur Herstellung von Arzneimitteln zur Senkung von ApoB-100 assoziierten Lipoproteinen.

Es ist bekannt, daß erhöhte Blutspiegel von Triglyceriden (Hypertriglyceridämie) und Cholesterin (Hypercholesterinämie) mit der Genese von atherosklerotischen Gefäßwand Verändungen und koronaren Herzkrankheiten assoziiert sind.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apolipoprotein B-100 einhergeht. Es ist daher nach wie vor ein starkes Bedürfnis, wirksame Arzneitmittel zur Bekämpfung der Atherosklerose sowie koronarer Herzkrankheiten zur Verfügung zu stellen.

Aus der Publikation EP-344 519 sind die Verbindungen 2(R*)- und 2(S*)-2-[4-(Chinolin-2-yl-methoxy)phenyl]-2-cyclopentylessigsäure-(S)-phenylglycinolamid bekannt.

Die WO 92/10468 und WO 92/09561 beschreiben anti-artheriosklerotisch wirkende Arylverbindungen bzw. Amide, die zu den Acyl-Coenzym-A-Cholesterol -Acyltransferase-Hemmern gehören. Strukturelle Ähnlichkeiten der dort offenbarten Verbindungen mit denen der vorliegenden Erfindung beschränken sich auf das Vorhandensein der gemeinsamen Strukturelemente eines Phenyl-Rests und einer Carbonylfunktion.

Die vorliegende Erfindung betrifft jetzt dir Vermendung von bicyclisch substituierten 0xy-phenyl-(phenyl)glycinolamiden der allgemeinen Formel (I),

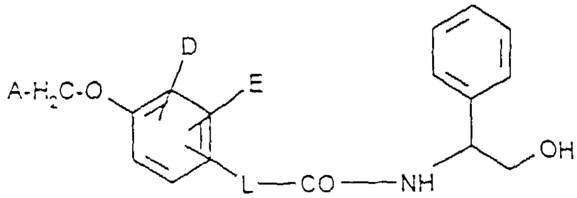 (I)

in welcher

A für Naphthyl
oder für einen Rest der Formel

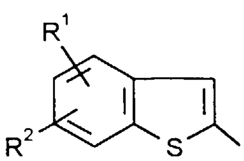 , 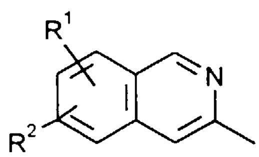 , 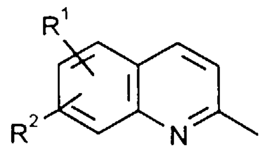 ,

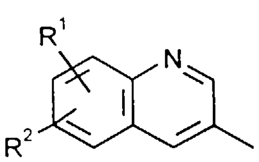 , 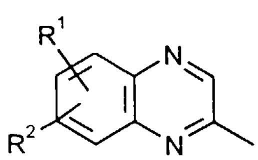 oder 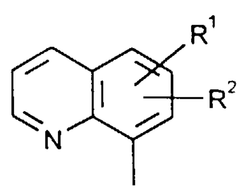

steht,
worin

$R^1$ und $R^2$ gleich oder verschieden sind und Wasserstoff, Halogen, Hydroxy oder geradkettiges oder verzweigtes Alkoxy mit bis zu 4 Kohlenstoffatomen bedeuten,

D und E gleich oder verschieden sind und für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen, einen 5- bis 6-gliedrigen ungesättigten oder gesättigten Heterocyclus mit bis zu 3 Heteroatomen aus der Reihe S, N und/oder O stehen,
oder
für einen Rest der Formel $-NR^3R^4$ oder $-NR^5SO_2-R^6$ stehen,
worin

$R^3$, $R^4$ und $R^5$ gleich oder verschieden sind und Wasserstoff oder geradkettiges oder verzweigtes Alkyl mit bis zu 4 Kohlenstoffatomen oder Phenyl bedeuten,

$R^6$ geradkettiges oder verzweigtes Alkyl mit bis zu 4 Kohlenstoffatomen, Benzyl oder Phenyl bedeutet, das gegebenenfalls durch geradkettiges oder verzweigtes Alkyl mit bis zu 4 Kohlenstoffatomen substituiert ist,

L für eine Bindung steht,
oder
für einen Rest der Formel

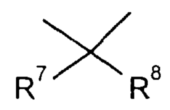

steht,
worin

$R^7$ Wasserstoff, Hydroxy, Methoxy oder Halogen bedeutet,

$R_8$ Wasserstoff, Hydroxy, Halogen, geradkettiges oder verzweigtes Alkenyl oder Alkoxy mit jeweils bis zu 8 Kohlenstoffatomen oder Cycloalkyl oder Cycloalkenyl mit jeweils 3 bis 14 Kohlenstoffatomen bedeutet, oder
geradkettiges oder verzweigtes Alkyl mit bis zu 8 Kohlenstoffatomen bedeutet, das gegebenenfalls durch Cycloalkyl mit 3 bis 8 Kohlenstoffatomen, Phenyl oder Tetrahydropyranyl substituiert ist, welche ihrerseits durch Halogen substituiert sein können,
oder einen Rest der Formel
$-CH_2SiR^9R^{10}R^{11}$ oder einen Indanylrest bedeutet,
worin

$R^9$, $R^{10}$ und $R^{11}$ gleich oder verschieden sind und geradkettiges oder verzweigtes Alkyl mit bis zu 4 Kohlenstoffatomen bedeuten

oder

$R^7$ und $R^8$ gemeinsam mit dem Kohlenstoffatom einen gesättigten carbocyclischen Ring mit 5 bis 7 Kohlenstoffatomen bilden, der gegebenenfalls bis zu 2-fach gleich oder verschieden durch geradkettiges oder verzweigtes Alkyl mit bis zu 4 Kohlenstoffatomen substituiert ist,

oder

$R^7$ und $R^8$ gemeinsam einen Rest der Formel = O oder einen Doppelbindungs-Rest der Formel

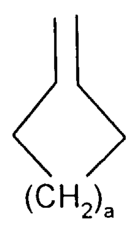

bilden,
worin

a eine Zahl 2, 3, 4, 5 oder 6 bedeutet,

und deren Salze, zur Herstellung von Arzneimitteln zur Senhung von ApoB-100-assoziirten Lipoproteinen.

Die erfindungsgemäß verwendeten bicyclisch substituierten Oxy-phenyl-(phenyl)glycinol-amide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe oder einen Tetrazolylrest besitzen, sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Heterocyclus steht im allgemeinen für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt sind 5-und 6-gliedrige Ringe mit einem Sauerstoff-, Schwefel- und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Ein 5- bis 6-gliedriger, gesättigter Heterocyclus, der außerdem als Heteroatome bis zu 3 Sauerstoff-, Schwefel- und/oder Stickstoffatome enthalten kann, steht im allgemeinen für Piperidyl, Morpholinyl, Piperazinyl oder Pyrrolidinyl. Bevorzugt ist Morpholinyl.

Carbocyclus steht im allgemeinen für einen 3- bis7-gliedrigen, bevorzugt 5-bis 7-gliedrigen gesättigten Kohlenwasserstoffring. Bevorzugt werden genannt Cyclopentyl, Cyclohexyl oder Cycloheptyl.

Die erfindungsgemäß verwendeten Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Beispielsweise werden folgende Diastereomere der Formeln (Ia) und (Ib) aufgeführt:

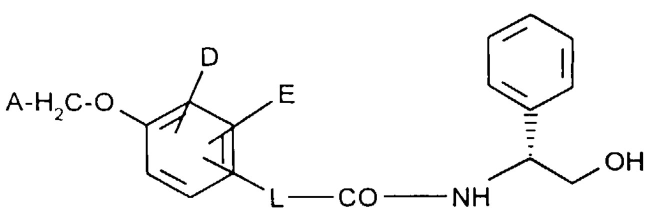

(Ia)

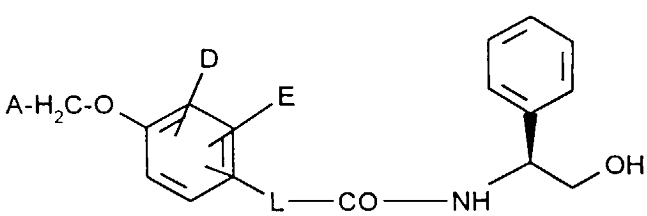

(Ib)

Bevorzugt sind Verbindungen der allgemeinen Formel (I),
in welcher

A für Naphthyl oder
für einen Rest der Formel

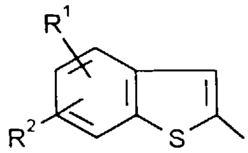 , 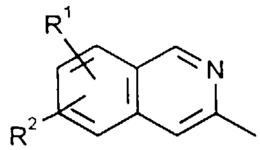 , 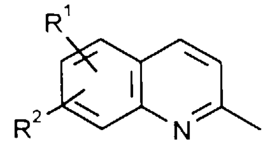 ,

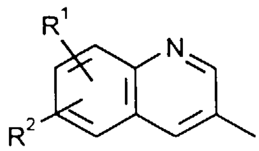 , 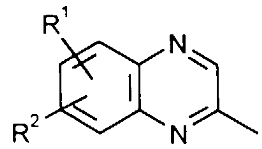 oder 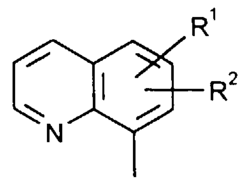

steht,
worin

$R^1$ und $R^2$ gleich oder verschieden sind und Wasserstoff, Fluor, Chlor, Brom, Hydroxy oder geradkettiges oder verzweigtes Alkoxy mit bis zu 3 Kohlenstoffatomen bedeuten,

D und E gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 5 Kohlenstoffatomen, Pyrryl oder Imidazolyl stehen,
oder
für einen Rest der Formel $-NR^3R^4$ oder-$NR^5$-$SO_2$-$R^6$ stehen,
worin

$R^3$, $R^4$ und $R^5$ gleich oder verschieden sind und Wasserstoff, geradkettiges oder verzweigtes Alkyl mit bis zu 3 Kohlenstoffatomen oder Phenyl bedeuten,

$R^6$ geradkettiges oder verzweigtes Alkyl mit bis zu 3 Kohlenstoffatomen, Benzyl oder Phenyl bedeutet, das gegebenenfalls durch geradkettiges oder verzweigtes Alkyl mit bis zu 3 Kohlenstoffatomen substituiert ist,

L für eine Bindung steht,
oder
für einen Rest der Formel

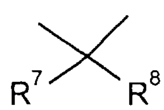

steht,
worin

$R^7$ Wasserstoff, Hydroxy, Methoxy, Fluor, Chlor oder Brom bedeutet,

$R^8$ Wasserstoff, Hydroxy, Halogen, geradkettiges oder verzweigtes Alkenyl oder Alkoxy mit jeweils bis zu 7 Kohlenstoffatomen, Cyclopentyl, Cyclohexyl, Cycloheptyl, Cyclooctyl, Cyclononyl, Cyclodecyl, Cycloundecyl, Cyclododecyl oder Cyclohexenyl bedeutet, oder

geradkettiges oder verzweigtes Alkyl mit bis zu 7 Kohlenstoffatomen bedeutet, das gegebenenfalls durch Cyclopentyl, Cyclohexyl, Cycloheptyl, Cyclooctyl, Phenyl oder Tetrahydropyranyl substituiert ist, welche ihrerseits durch Fluor, Chlor oder Brom substituiert sein können, oder einen Rest der Formel $CH_2$-$SiR^9R^{10}R^{11}$ oder einen Indanylrest bedeutet,
worin
$R^9$, $R^{10}$ und $R^{11}$ gleich oder verschieden sind und geradkettiges oder verzweigtes Alkyl mit bis zu 3 Kohlenstoffatomen bedeuten

oder

$R^{13}$ und $R^{14}$ gemeinsam mit dem Kohlenstoffatom einen Cyclopentyl-, Cyclohexyl- oder Cycloheptylring bilden, die gegebenenfalls bis zu 2-fach durch Methyl substituiert sind

oder

$R^7$ und $R^8$ gemeinsam einen Rest der Formel = O oder einen Doppelbindungs-Rest der Formel

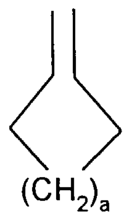

bilden,
worin

a eine Zahl 2,3 oder 4 bedeutet,

und deren Salze.

Besonders bevorzugt sind Verbindungen der allgemeinen Formel (I),
in welcher

A für Naphtyl
oder
für einen Rest der Formel

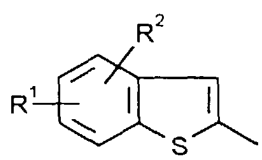 , 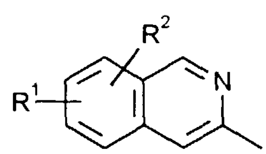 , 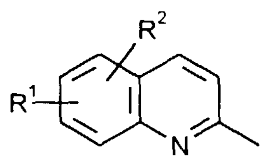 ,

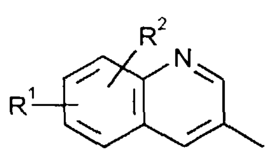 , 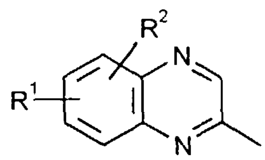 oder 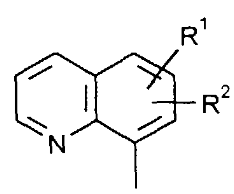

steht,
worin

$R^1$ und $R^2$ gleich oder verschieden sind und Wasserstoff, Fluor, Chlor, Brom, Hydroxy oder Methoxy bedeuten,

D und E gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 5 Kohlenstoffatomen, Pyrryl oder Imidazolyl stehen,
oder
für einen Rest der Formel $-NR^5-SO_2-R^6$ stehen,
worin

$R^5$ Wasserstoff oder Methyl bedeutet,
$R^6$ geradkettiges oder verzweigtes Alkyl mit bis zu 3 Kohlenstoffatomen, Benzyl oder Phenyl bedeutet, das gegebenenfalls durch Methyl oder Ethyl substituiert ist,

L für eine Bindung steht,
oder
für einen Rest der Formel

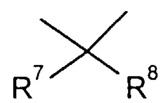

steht,
worin

$R^7$ Wasserstoff, Hydroxy, Fluor oder Methoxy bedeutet,
$R^8$ Wasserstoff, Hydroxy, geradkettiges oder verzweigtes Alkenyl oder Alkoxy mit jeweils bis zu 6 Kohlenstoffatomen, Cyclopentyl, Cyclohexyl, Cycloheptyl, Cyclooctyl, Cyclononyl, Cyclodecyl, Cycloundecyl, Cyclododecyl oder Cyclohexenyl bedeutet, oder geradkettiges oder verzweigtes Alkyl mit bis zu 6 Kohlenstoffatomen bedeutet, das gegebenenfalls durch Cyclopentyl, Cyclohexyl, Cycloheptyl, Phenyl oder Tetrahydropyranyl substituiert ist, welche ihrerseits durch Fluor, Chlor oder Brom substituiert sein können, oder einen Rest der Formel $CH_2Si(R^9R^{10}R^{11})$ oder einen Indanylrest bedeutet,
worin
$R^9$, $R^{10}$ und $R^{11}$ Methyl bedeuten

oder

$R^7$ und $R^8$ gemeinsam mit dem Kohlenstoffatom einen Cyclopentyl-, Cyclohexyl- oder Cycloheptylring bilden, die gegebenenfalls bis zu 2-fach durch Methyl substituiert sind

oder

$R^7$ und $R^8$ gemeinsam einen Rest der Formel = 0 oder einen Doppelbindungs-Rest der Formel

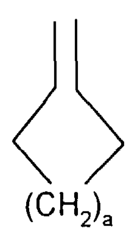

bedeuten,
worin

a eine Zahl 2, 3 oder 4 bedeutet,

und deren Salze.

Außerdem wurde ein Verfahren zur Herstellung der Verbindungen der allgemeinen Formel (I) gefunden, dadurch gekennzeichnet, daß man Carbonsäuren der allgemeinen Formel (II)

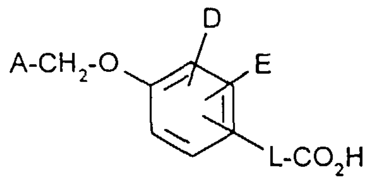

(II)

in welcher
A, D, E und L die angegebene Bedeutung haben,
gegebenenfalls unter vorgeschalteter Aktivierung der Carbonsäurefunktion mit Phenylglycinol der Formel (III)

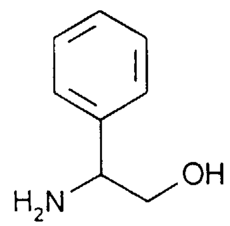

(III)

gegebenenfalls unter Schutzgasatmosphäre, gegebenenfalls in inerten Lösemitteln, in Anwesenheit einer Base und/oder eines Hilfsmittels umsetzt,
und im Fall $R^3$, $R^4$ und/oder $R^5 \neq$ H gegebenenfalls eine Alkylierung anschließt,
und gegebenenfalls die Substituenten $R^1$, $R^2$, D und E variiert.

Das Verfahren kann durch folgendes Formelschema beispielhaft erläutert werden:

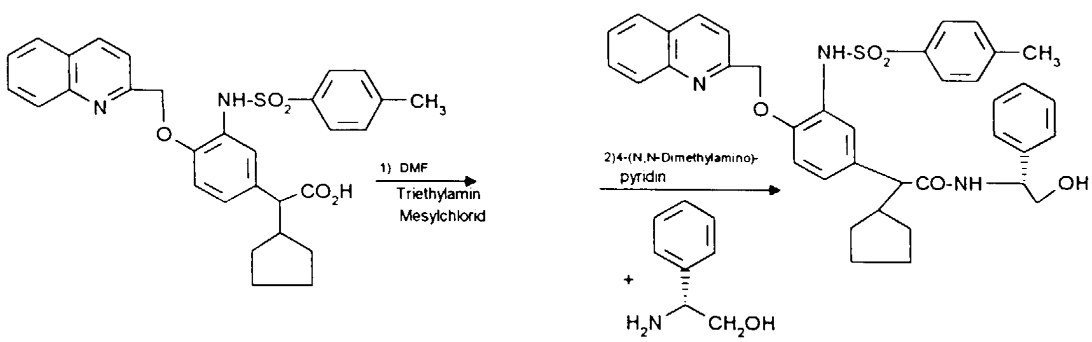


Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohenwasserstoffe wie Benzol, Xylol,Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton oder Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliumethanolat, oder Natrium- oder Kaliummethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat und Triethylamin.

Die Base wird in einer Menge von 0,6 mol bis 5 mol, bevorzugt von 0,7 mol bis 2 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Zur Aktivierung der Carbonsäurefunktion eignen sich im allgemeinen Basen und/oder Dehydratisierungsreagenzien wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Aktivierung der Carbonsäurefunktion erfolgt im allgemeinen in einem Temperaturbereich von 0°C bis 160°C, bevorzugt von 30°C bis 80°C und gegebenenfalls Schutzgasatmosphäre.

Die Verbindungen der allgemeinen Formel (II) sind bekannt (vgl. EP 582 908, 344 519, 339 416, 399 291, 414 078, 529 450; und 414 076; US 4 929 629, 4 970 215, 5 091 392, 5 126 354, DE 410 555.1, 4 112 533.9, 41 128 681.2, 4 219 765.1, 4 226 519.3, 4 226 649.1) oder können hergestellt werden, indem man

Verbindungen der allgemeinen Formel (IV)

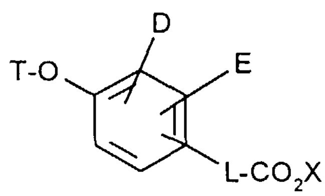

(IV)

in welcher

D, E und L die angegebene Bedeutung haben,
T für eine typische Hydroxyschutzgruppe, bevorzugt für Benzyl oder tert.-Butyl steht,

und

X für Wasserstoff oder für ($C_1$-$C_4$)-Alkyl steht

nach Abspaltung dieser Schutzgruppe nach üblichen Methoden,
mit Verbindungen der allgemeinen Formel (V)

$$A\text{-}CH_2\ Z \qquad (V)$$

in welcher

A die oben angegebene Bedeutung hat

und

Z für Halogen, vorzugsweise Brom steht,

in inerten Lösemitteln, gegebenenfalls in Anwesenheit einer Base umsetzt,
und im Fall der Säuren die Ester verseift.

Die Verbindung der allgmeinen Formel (III) ist bekannt.

Die Verbindungen der allgemeinen Formel (IV) und (V) sind an sich bekannt oder nach üblichen Methoden herstellbar.

Die Verbindungen der allgemeinen Formel (I) haben ein nicht vorhersehbares pharmakologisches Wirkspektrum.

Ebenso zeigen die bekannten Verbindungen 2(R*)- und 2(S*)-2-[4-Chinolin-2-yl-methoxy)phenyl]-2-Cyclopentylessigsäure(S)phenylglycinolamid neben ihrer Lipoxygenashemmenden Wirkung dieses neue pharmakologische Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die Verbindungen zeichnen sich durch eine Senkung der ApoB-100-assoziierten Lipoproteinen (VLDL und seiner Abbauprodukte, wie LDL), des ApoB-100, der Trigylceriden und des Cholesterins aus. Damit besitzen sie wertvolle, im Vergleich zum Stand der Technik überlegene pharmakologische Eigenschaften.

Überraschenderweise besteht die Wirkung der Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der oben genannten Risikofaktoren gesenkt, die an Gefäßwandveränderungen beteiligt sind.

Die Verbindungen können deshalb zur Präventation und Behandlung von Atherosklerose, Fettsucht, Pankreatitis und der Obstipation eingesetzt werden.

1. Hemmung der Freisetzung ApoB-100-assoziierter Lipoproteine

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL-bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten (Nunc). Wenn im Überstand von HepG2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase (Boehringer, Mannheim) konjugiert. Peroxidase setzt das farblose Substrat TMB (Kirkegaard u. Perry) in Gegenwart von $H_2O_2$ in ein gefärbtes Produkt um. Nach Ansäuerung des Reaktionsgemisches mit $H_2SO_4$ wird die spezifische Lichtabsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die Verbindungen die Freisetzung der ApoB-100-assoziierten Partikel. Der $IC_{50}$-Wert gibt an, bei welcher Substanzkonzentration die Lichtabsorption im Vergleich zur Kontrolle (Lösemittelkontrolle ohne Substanz) um 50% inhibiert ist.

2) Untersuchung zur Inhibition der Proliferation glatter Muskelzellen

Zur Feststellung der antiproliferativen Wirkung der Verbindungen werden glatte Muskelzellen verwendet, die aus den Aorten von Ratten durch die Media--Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt. Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

3) Bestimmung der VLDL-Sekretion in vivo (Hamster)

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i. p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentriugiert. Im so erhaltenen Serum wird dieTriglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

4) Hemmung der intestinalen Triglyceridabsorption in vivo (Ratte)

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird Ihnen das Futter entzogen. Trinkwaser steht den Tieren ad libitum zur Verfügung. Die Tiere der Kontrollgruppe erhalten eine wäßrige Traganth-Suspension bzw. eine Traganth-Suspension, die Olivenöl enthält. Die Traganth-Olivenöl-Suspension wird mit einem Ultra-Turrax hergestellt. Die zu untersuchenden Substanzen werden in einer entsprechenden Traganth-Olivenöl-Suspension ebenfalls mit einem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspensionen, die Traganth-Olivenöl-Suspensionen, ohne Substanz (Kontrolltiere) bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 oder 3 Stunden nach der Schlundsondenapplikation.

Die Blutproben werden zentrifugiert und nach Gewinnung des Serums die Triglyceride photometrisch mit einem EPOS-Analyzer 5060 (Eppenford Gerätebau, Netheler & Hinz GmbH, Hamburg) bestimmt. Die Bestimmung der Triglyceride erfolgen vollenzymatisch mit einem handelsüblichen UV-Test.

Der postprandiale Serumtriglyceridanstieg wird durch Subtraktion des Triglyceridvorwertes jedes Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridantiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{Substanz} - \Delta TG_{Traganthkontrolle}}{\Delta TG_{Ölbelastung} - \Delta TG_{Traganthkontrolle}} \times 100$$

Effekt von 10 mg Prüfsubstanz / kg KG p.o. auf den Triglyceridanstieg Δ(%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0,05$) um mindestens 30 % vermindern, werden als pharmakologisch wirksam angesehen.

5) Hemmung der VLDL-Sekretion in vivo (Ratte)

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339 gelöst in physiologischer Kochsalzlösung intravenös in die Schwanzvene appliziert. Triton WR-1339 inhibiert die Lipoproteinlipase und führt somit durch Hemmung des VLDL-Katabolismus zu einem Anstieg des Triglycerid- und Cholesterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subkutan appliziert.

Erfindungsgemäß kann außerdem die Kombination von bicyclisch substituierten Oxy-phenyl-(phenyl)glycinolamiden der allgemeinen Formel (I) mit einem Glucosidase- und/oder Anylasehemmer zur Behandlung von familiären Hyperlipidämien, der Fettsucht (Adiposilas) und des Diabetes mellitus, verwendetwerden. Glucosidasen und/oder Arylasehemmer im Rahmen der Erfindung sind beispielsweise Acarbose, Adipisine, Voglibose (AO-128), Miglitol, Emiglitate, MDL-25637, Camiglibase (MDL-73945), Tendamistate, AI-3688, Trestatin, Pradimicin-Q und Salbostatine.

Bevorzugt ist die Kombination von Acarbose, Miglitol, Emiglitate oder Voglibase mit einer der oben aufgeführten erfindungsgemäßen Verbindungen der allgemeinen Formel (I).

Die Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/ oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1mg/kg, vorzugsweise etwa 0,01 bis 0,5mg/kg Körpergewicht zur Erzielung wirksamer Ergebnisse zu verabreichen, und bei oraler Applikation beträgt die Dosierung etwa 0,01 bis 20mg/kg, vorzugsweise 0,1 bis 10mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

| **Benutzte Solvenzgemische:** | | | |
|---|---|---|---|
| A | $\triangleq$ | Petrolether : Aceton = | 1:1 |
| B | $\triangleq$ | Petrolether : Essigester = | 1:1 |
| C | $\triangleq$ | Dichlormethan : Methanol = | 10:1 |
| D | $\triangleq$ | Dichlormethan : Essigester = | 1:1 |
| E | $\triangleq$ | Dichlormethan : Methanol = | 20:1 |
| F | $\triangleq$ | Dichlormethan : Ethanol = | 50:1 |
| G | $\triangleq$ | Dichlormethan : Methanol = | 50:1 |

(fortgesetzt)

| Benutzte Solvenzgemische: | | | |
|---|---|---|---|
| H | △ | Dichlormethan : Essigester = | 5:1 |

**Verwendete Abkürzungen:**

| | |
|---|---|
| Ac = | Acetyl |
| Bn = | Benzyl |
| Bz = | Benzoyl |
| iBu = | iso Butyl |
| nBu = | normal Butyl |
| sBu = | sekundär Butyl |
| tBu = | tertiär Butyl |
| cDec = | cyclo-Decyl |
| DMF = | N,N-Dimethylformamid |
| DMSO = | Dimethylsulfoxid |
| cDodec = | cyclo-Dodecyl |
| Et = | Ethyl |
| cHept = | cyclo-Heptyl |
| cHex = | cyclo-Hexyl |
| HOBT = | 1 -Hydroxy -1H -benzotriazol |
| Me = | Methyl |
| Mes = | Mesyl |
| cNon = | cyclo-Nonyl |
| cOct = | cyclo-Octyl |
| cPent = | cyclo-Pentyl |
| nPent = | normal Pentyl |
| Ph = | Phenyl |
| cPr = | cyclo-Propyl |
| nPr = | normal Propyl |
| iPr = | iso-Propyl |
| THF = | Tetrahydrofuran |
| TMS = | Tetramethylsilan |
| pTol = | para-Tolyl |
| pTos = | para-Tosyl |
| cUndec = | cyclo-Undecyl |

**Definition der Isomerentypen:**

| | |
|---|---|
| 4dia = | Gemisch der vier möglichen Diasteromeren bei zwei Asymmetriezentren im Molekül |
| dia A = | Diastereomer mit dem größeren $R_f$-Wert |
| dia B = | Diastereomer mit dem kleineren $R_f$-Wert |
| ent = | Enantiomer |
| 2 ent dia = | Gemisch zweier enantiomerenreiner Diastereomere |
| ent dia A = | enatiomerenreines Diastereomer mit dem größeren $R_f$-Wert |
| ent dia B = | enatiomerenreines Diastereomer mit dem kleineren $R_f$-Wert |

R = R-Enantiomer

rac = Racemat

rac dia A = racemisches Diastereomer mit dem größeren $R_f$-Wert

rac dia B = racemisches Diastereomer mit dem kleineren $R_f$-Wert

S = S-Enantiomer

**Ausgangsverbindungen**

**Beispiel I**

Cyclononanol

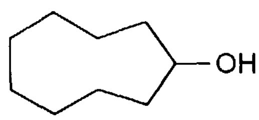

25,3 g Cyclononanon werden in 200 ml wasserfreiem Ether gelöst und bei Raumtemperatur mit 10,25 g Lithiumalanat umgesetzt. Nach 1,5 Stunden hydrolysiert man vorsichtig mit einer wäßrigen Natriumchloridlösung und extrahiert dann mit Ether/wäßriger Zitronensäure. Die organische Phase wird mit Magnesiumsulfat getrocknet und im Vakuum eingedampft.
Ausbeute: 25,6 g
$R_f$ = 0,26 (Dichlormethan)
charakteristisches $^1$H-NMR-Signal ($CDCl_3$, 250 MHz, TMS): $\delta$ = 3,88 (m, 1H, C<u>H</u>-OH) ppm.

**Beispiel II**

Cycloundecanol

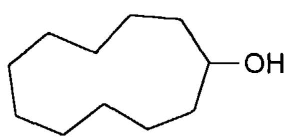

Analog der Vorschrift für Beispiel I werden 9,6 g Cycloundecanon umgesetzt. Ausbeute: 9,3 g
$R_f$ = 0,28 (Dichlormethan)
charakteristisches $^1$H-NMR-Signal ($CDCl_3$, 250 MHz, TMS): $\delta$ = 3,88 (m, 1H, C<u>H</u>-OH).

**Beispiel III**

Cyclononylbromid

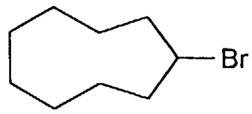

5,0 g der Verbindung aus Beispiel I werden 6 Stunden mit 12 ml 48 %iger Bromwasserstoffsäure und 2,8 ml konzentrierter Schwefelsäure bei Raumtemperatur gerührt. Darauf setzt man Dichlormethan zu und wäscht die organische Phase mehrfach mit Wasser bis die wäßrige Phase nach dem Ausschütteln einen pH > 5 besitzt. Die mit Magnesiumsulfat getrocknete Dichlormethanlösung wird im Vakuum eingedampft. Das so erhaltene Rohprodukt enthält laut $^{1}H$-NMR kein Edukt, allerdings findet sich in dem rohen Produkt Z-Cyclononen. Gesamtausbeute: 5,1 g
molares Verhältnis Cyclononylbromid : Z-Cyclononen 8,8 :1 charakteristiche $^{1}H$-NMR-Signale ($CDCl_3$, 250 MHz, TMS):

Cyclononylbromid: $\delta$ = 4,41 ppm (m, 1H, C<u>H</u>Br)
Z-Cyclononen: $\delta$ = 5,53 ppm (m, 2H, C<u>H</u>=C<u>H</u>)

**Beipiel IV**

Cycloundecylbromid

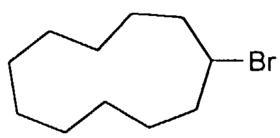

Die Umsetzung wird mit 2,6 g der Verbindung aus Beispiel II analog der Vorschrift für Beispiel III durchgeführt. Ausbeute: 2,5 g Rohprodukt
charakteristisches $^{1}H$-NMR-Signal ($CDCl_3$, 250 MHz, TMS): $\delta$ = 4,36 ppm (m, 1H, C<u>H</u>Br).

Das rohe Cycloundecylbromid wird mit den olefinischen Nebenprodukten E-und Z-Olefin weiter umgesetzt.

**Beispiel V**

2-[4-(Chinolin-2-yl-methoxy)phenyl]-2-cyclononyl-essigsäuremethylester

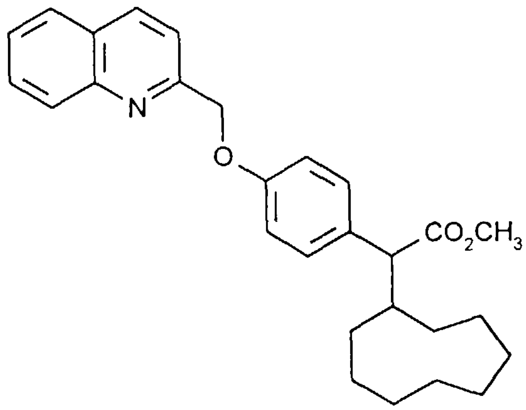

7,11 g 2-[4-(Chinolin-2-yl-methoxy)phenyl]essigsäuremethylester (US 4 970 215) werden in 50 ml wasserfreiem DMF bei 0°C mit 3,23 g Kalium-tert.butanolat in 40 ml wasserfreiem DMF verrührt, 1 h bei 0°C nachgerührt und dann mit 5,1 g der Verbindung aus Beispiel III versetzt. Die Mischung wird insgesamt 20 Stunden unter allmählicher Erwärmung auf Raumtemperatur nachgerührt, auf Wasser und Ether gegossen und mit 2 M Salzsäure auf pH ≈ 4 gestellt. Die organische Phase wird abgetrennt, mit Magnesiumsulfat getrocknet und eingedampft. Der so erhaltene Rohprodukt wird säulenchromatographisch gereinigt (Kieselgel 60, Merck 40-63 µm, Petrolether : Essigester = 10:1). Neben reisoliertem Edukt fallen 2,8 g Produkt an.
$R_f$ = 0,48 (Petrolether : Essigester = 1:1)

**Beispiel VI**

2-[4-(Chinolin-2-yl-methoxy)phenyl]-2-cycloundecyl-essigsäuremethylester

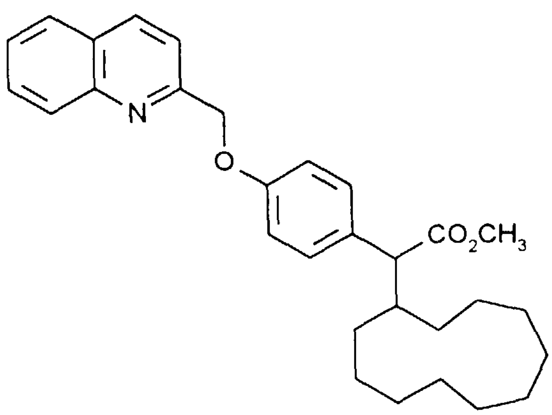

Die Reaktion wird mit 2,5 g der Verbindung aus Beispiel IV und 3,06 g 2-[4-(Chinolin-2-yl-methoxy)phenyl] essigsäuremethylester (US 4 970 215) analog der Vorschrift für Beispiel V durchgeführt.
Ausbeute: 0,72 g
$R_f$ = 0,41 (Petrolether : Essigester = 5:1)

**Beispiel VII**

2-[4-(Chinolin-2-yl-methoxy)phenyl]-2-cyclononyl-essigsäure

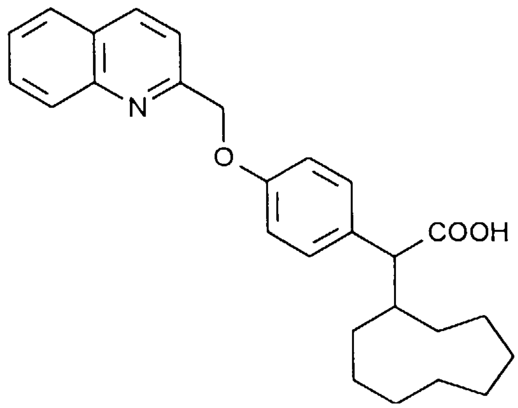

2,06 der Verbindung aus Beispiel V werden in 40 ml Ethanol 10 Stunden mit 20 ml 2 M wäßriger Natronlauge unter Rückfluß gekocht. Die abgekühlte Reaktionsmischung wird in Ether/Wasser gegossen, die alkalische wäßrige Phase mit 1 M Salzsäure auf pH ≈ 2 gestellt und wiederum mit Ether extrahiert. Die letztgenannte organische Phase wird mit Magnesiumsulfat getrocknet, und im Vakuum bis zur Trockene eingedampft.
Ausbeute: 0,3 g
$R_f$ = 0,04 (Petrolether : Essigester = 5:1)

**Beispiel VIII**

2-[4-(Chinolin-2-yl-methoxy)phenyl]-2-cycloundecyl-essigsäure

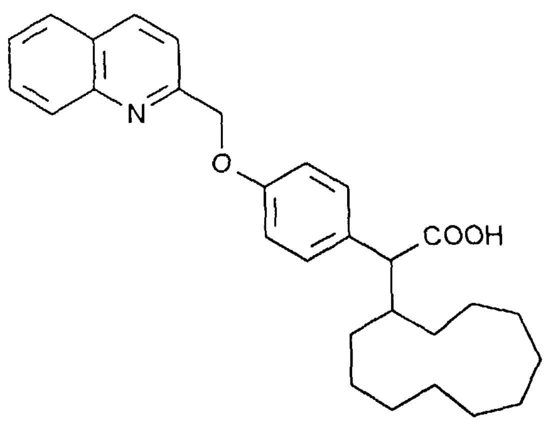

Die Umsetzung wird mit 0,5 g der Verbindung aus Beispiel VI analog der Reaktionsvorschrift von Beispiel VII durchgeführt.
Ausbeute: 0,46 g
$R_f$= 0,37 (Dichlormethan : Methanol = 20:1)

**Beispiel IX**

2-[3-Amino-4-(chinolin-2-yl-methoxy)phenyl]-2-cyclopentylessigsäuremethylester

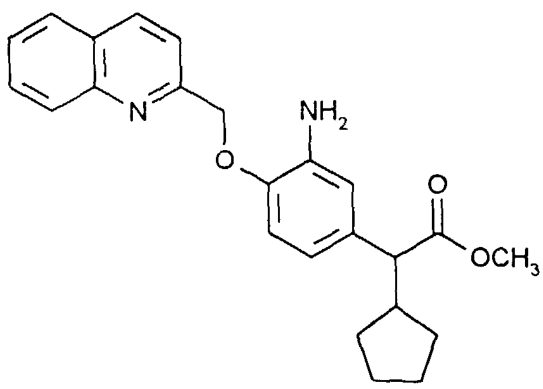

1 g 2-[3-Nitro(-4-(chinolin-2-yl-methoxy)phenyl]-2-cyclopentylessigsäuremethylester (2,4 mmol; aus DE 41 05 551) wird in THF (5 ml) und Methanol (20 ml) gelöst. Triethylamin (0,486 g, 4,8 mmol) und 1,3-Dimercaptopropan (0,52 g, 4,8 mmol) werden tropfenweise dazugegeben. Nach 18 h wird die Reaktionsmischung mit 1 M wäßriger HCl (20 ml) versetzt. Die Lösung wird einrotiert, und der Rückstand in Diethylether und Wasser gelöst. Die wäßrige Phase wird mit $NaHCO_3$ neutralisiert, und mit Diethylether (3 mal) extrahiert. Die Diethylether-Phase wird getrocknet und einrotiert.
Ausbeute: 0,75 g (80%)
$R_f$= 0,31(100:2 = $CH_2Cl_2$: $CH_3OH$)
MS (DCI, $NH_3$): 391 ($[M+H]^+$)
IR (Film): $\tilde{\nu}$ = 3434, 3312, 2951, 1735, 1518 $cm^{-1}$

**Beispiel X**

2-[3-(N-Mesyl)amino-4-(chinolin-2-yl-methoxy)phenyl]-2-cyclopentyl-essigsäure--methylester

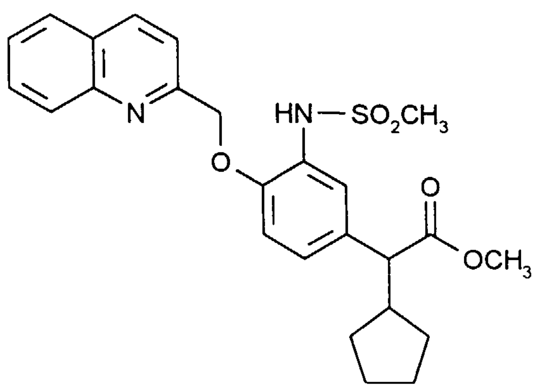

1,2 g der Verbindung aus Beispiel IX (3,0 mmol) werden in 12 ml $CH_2Cl_2$ gelöst und auf 0°C gekühlt. 0,45 g Methansulfonsäurechlorid (4,0 mmol) in 4 ml $CH_2Cl_2$ wird tropfenweise dazugegeben. Nach 10 min wird mit Triethylamin (0,37 g, 3,7 mmol) versetzt und auf 25°C erwärmt. Nach 3 h werden weitere 15 ml $CH_2Cl_2$ dazugegeben, und die Lösung mit 1 m wäßriger HCl sowie mit gesättigter wäßriger $NaHCO_3$-Lösung gewaschen. Die Lösung wird getrocknet ($Na_2SO_4$) und einrotiert. Der Rückstand wird säulenchromatographisch getrennt.
Ausbeute: 1,22 g (85%)
Rf= 0,47 (100:2 = $CH_2Cl_2$: $CH_3OH$)

**Beispiel XI**

2-(3-Pyrrolyl-4-(chinolin-2-yl-methoxy)phenyl]-2-cyclopentylessigsäuremethylester

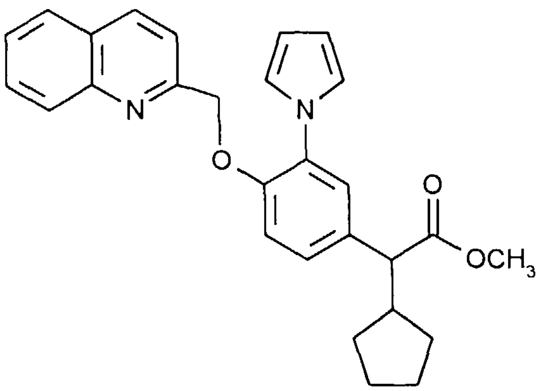

1,0 g der Verbindung aus Beispiel IX (2,56 mmol) werden in 15 ml Essigsäure gelöst, mit 0,42 g 2,5-Dimethoxytetrahydrofuran (3,2 mmol) versetzt und 3 h zum Sieden erhitzt. Nach Abdestillieren der Essigsäure im Vakuum, Aufnehmen in $CH_2Cl_2$, Ausschütteln mit Wasser, Trocknen ($Na_2SO_4$) und Einengen im Vakuum wird der Rückstand säulenchromatographisch getrennt.
Ausbeute: 0,62 g (55%)
$R_f$= 0,69 (100:2 = $CH_2Cl_2$: $CH_3OH$)
MS (EI): 440 ($M^+$)

**Beispiel XII**

2- [3-(N-Tosyl)amino-4-(chinolin-2-yl-methoxy)phenyl]-2-cyclopentylessigsäure-methylester

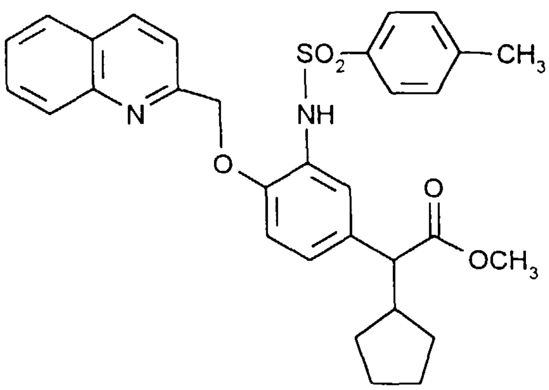

1,2 g der Verbindung aus Beispiel IX (3,1 mmol) werden in 15 ml $CH_2Cl_2$ gelöst und auf 0°C gekühlt. Dann versetzt man tropfenweise mit 0,76 g p-Toluolsulfonsäurechlorid (4,0 mmol) in 4 ml $CH_2Cl_2$. Nach 10 min werden 0,37 g Triethylamin (3,7 mmol) dazugegeben, und man erwärmt auf 25°C. Nach 2 h werden weitere 15 ml $CH_2Cl_2$ zugesetzt und die Lösung mit 1 M wäßriger HCl sowie mit gesättigter wäßriger $NaHCO_3$-Lösung gewaschen. Die Lösung wird getrocknet ($Na_2SO_4$) und einrotiert. Der Rückstand wird säulenchromatographisch gereinigt. Ausbeute: 1,07 g (64%)
Rf= 0,27 (100:2 = $CH_2Cl_2$: $CH_3OH$)
MS (FAB): 545 (M+H)

Beispiel XIII

2-[3-(N-Mesyl)amino-4-(chinolin-2-yl-methoxy)phenyl]-2-cyclopentylessigsäure

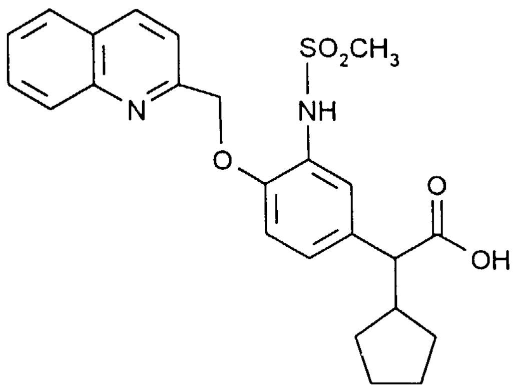

1,14 g der Verbindung aus Beispiel X (2,4 mmol) werden in Methanol (10 ml) gelöst, und mit 2 M Natronlauge (2,43 ml) versetzt. Nach 18 h wird die Lösung mit 1 M wäßriger HCl neutralisiert und dann abrotiert. Der Rückstand wird in 15 ml $CH_2Cl_2$ gelöst und mit Wasser gewaschen. Die Lösung wird getrocknet ($Na_2SO_4$) und einrotiert. Der Rückstand wird säulenchromatographisch getrennt.
Ausbeute: 0,66 g (60%)
$R_f$= 0,36 (100:5 = $CH_2Cl_2$:$CH_3OH$)

**Beispiel XIV**

2-[3-Pyrryl-4-(chinolin-2-yl-methoxy)phenyl]-2-cyclopentylessigsäure

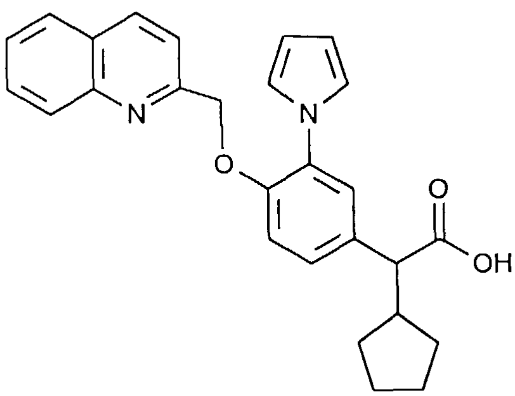

0,57 g der Verbindung aus Beispiel XI (1,3 mmol) werden in Methanol (6 ml) gelöst und mit 2 M Natronlauge (1,3 ml) versetzt. Nach 18 h wird die Lösung mit 1 M wäßriger HCl neutralisiert und abrotiert. Der Rückstand wird in 15 ml $CH_2Cl_2$ gelöst und mit Wasser gewaschen. Die Lösung wird getrocknet ($Na_2SO_4$) und einrotiert. Der Rückstand wird säulenchromatographisch getrennt.
Ausbeute: 0,48 g (85%)
$R_f$ = 0,36 (100:5 = $CH_2Cl_2$ : $CH_3OH$)

**Beispiel XV**

2-[3-(N-Tosyl)-amino-4-(chinolin-2-yl-methoxy)phenyl]-2-cyclopentylessigsäure

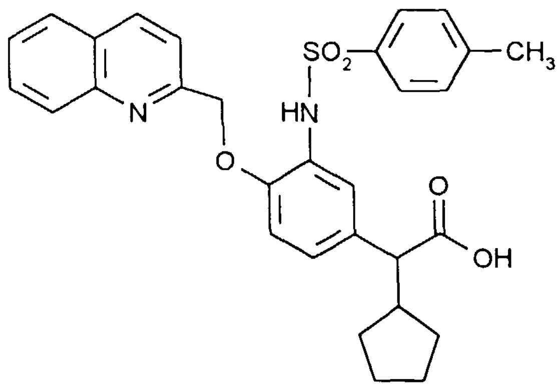

0,99 g der Verbindung aus Beispiel XII (1,8 mmol) werden in Methanol (10 ml) gelöst und mit 2 M Natronlauge (7,3 ml) versetzt. Nach 18 h wird die Lösung mit 1 M wäßriger HCl neutralisiert und abrotiert. Der Rückstand wird in 15 ml $CH_2Cl_2$ gelöst und mit Wasser gewaschen. Die Lösung wird getrocknet ($Na_2SO_4$) und einrotiert. Der Rückstand wird säulenchromtographisch getrennt.
Ausbeute: 0,90 g (94%)
$R_f$ = 0,27 (100:5 = $CH_2Cl_2$:$CH_3OH$)
Fp. = 117°C

**Beispiel XVI**

1,1-Dimethoxy-1-(2-fluor-4-methoxyphenyl)-2-jod-ethan

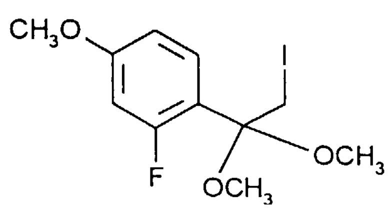

1,68 g 2-Fluor-4-methoxyacetophenon, 5,3 g Trimethylorthoformiat und 3,0 g Jod werden bei Raumtemperatur gemischt. Nach 18 h, wird eine 10 %ige wäßrige $Na_2S_2O_3$-Lösung dazugegeben, und mit Essigester extrahiert. Die organische Phase wird getrocknet ($Na_2SO_4$) und einrotiert. Der Rückstand wird säulenchromatographisch getrennt.
Ausbeute: 1,7 g (86%)
$R_f$ = 0,6 (1:5 = Petrolether : $CH_2Cl_2$)
MS (FAB): 340 ($M^+$), 309, 199

**Beispiel XVII**

2-(2-Fluor-4-methoxyphenyl)-essigsäuremethylester

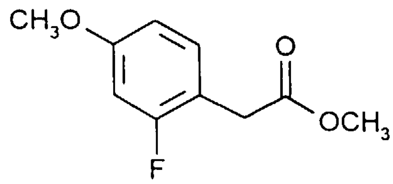

8,9 g der Verbindung aus Beispiel XVI und 27,7 g Trimethylorthoformiate werden in 26 ml Methanol gelöst. Unter Lichtausschluß werden 5,0 g Silbertetrafluoroborat dazugegeben. Nach 14 h wird die Lösung mit Wasser und Diethylether verdünnt. Die Phasen werden getrennt, und die organische Lösung wird getrocknet ($Na_2SO_4$) und einrotiert. Der Rückstand wird säulenchromatographisch getrennt. Ausbeute: 5,0 g (96%)
$R_f$ = 0,3 (10:1 = Petrolether : Essigester)
MS (FAB): 340 ($M^+$), 309, 199

**Beispiel XVIII**

2-(2-Fluor-4-methoxyphenyl)-2-cycloheptylessigsäuremethylester

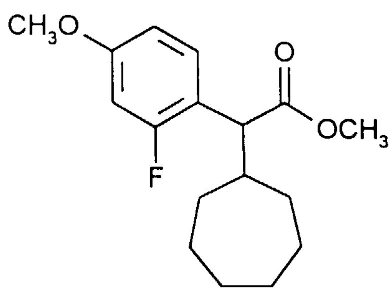

1,5 g NaH (60 %ig in Parafin) werden in 20 ml DMF gelöst und auf 0°C gekühlt. 5 g der Verbindung aus Beispiel XVII und 5,4 g Cycloheptylbromid werden in 20 ml DMF tropfenweise dazugegeben. Die Lösung wird auf 25°C erwärmt, und mit Wasser und Essigester verdünnt. Die Phasen werden getrennt, und die Lösung wird mit Wasser gewaschen, getrocknet ($Na_2SO_4$) und einrotiert. Der Rückstand wird säulenchromatographisch getrennt.
Ausbeute: 5,3 g (71%)
$R_f$ = 0,5 (10:1 = Petrolether : Essigester)
MS (DCI, $NH_3$): 312 ($M^+$+ $NH_3$), 295 ($M^+$), 212, 198, 139

**Beispiel XIX**

2-(2-Fluor-4-hydroxyphenyl)-2-cycloheptylessigsäureethylester

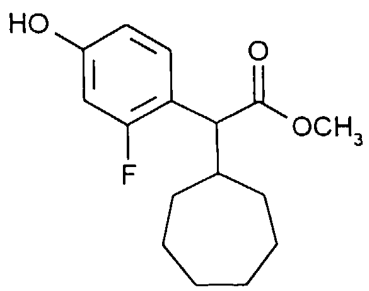

4 g der Verbindung aus Beispiel XVIII werden in 20 ml $CH_2Cl_2$ gelöst. Die Lösung wird auf -78°C gekühlt, und mit 100 ml Bortribromid versetzt. Die Lösung wird auf -30°C und nach einer Stunde auf 0°C erwärmt. Nach einer Stunde wird die Lösung auf -30°C gekühlt und mit 120 ml Methanol versetzt. Die Lösung wird mit Wasser und Essigester verdünnt. Die Phasen werden getrennt, die organische Lösung getrocknet ($Na_2SO_4$) und einrotiert. Der Rückstand wird säulenchromatographisch getrennt.
Ausbeute: 4,0 g (100%)
$R_f$= 0,25 (10:1 = $CH_2Cl_2$: Essigester)

**Beispiel XX**

2-[2-Fluor-4-(chinolin-2-yl-methoxy)phenyl]-2-cycloheptylessigsäuremethylester

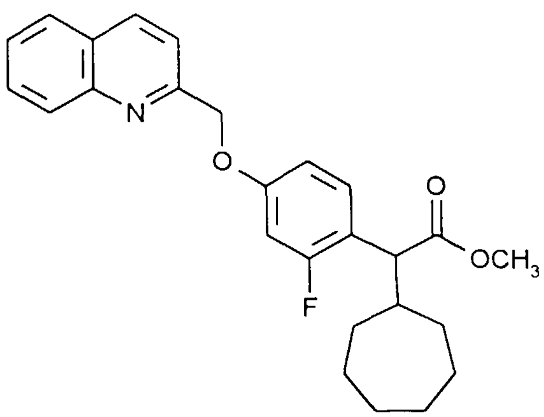

4,0 g der Verbindung aus Beispiel XIX werden in 40 ml DMF gelöst und tropfenweise zu 0,68 g Natriumhydrid (60 %ig in Parafin) in 8 ml DMF gegeben. Nach 30 Minuten werden 2,8 g 2-Chlormethylchinolin in 28 ml DMF tropfenweise zugegeben. Die Lösung wird auf 25°C erwärmt und mit Wasser und Essigester verdünnt. Die Phasen werden getrennt, die organische Lösung mit Wasser gewaschen, getrocknet ($Na_2SO_4$) und einrotiert. Der Rückstand wird säulenchromatographisch getrennt.
Ausbeute: 5,0 g (87%)
$R_f$ = 0,66 (Petrolether : Essigester = 3:1)
MS (FAB): 422 ($M^+$)

**Beispiel XXI**

2-[2-Fluor-4-(chinolin-2-yl-methoxy)phenyl]-2-cycloheptylessigsäure

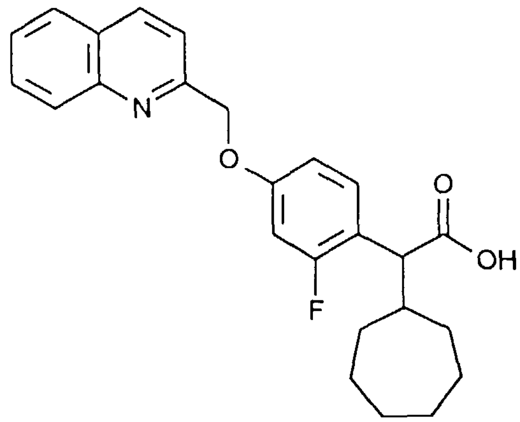

5,0 g der Verbindung aus Beispiel XX werden in 30 ml Methanol gelöst und 17,8 ml 2 M NaOH dazugegeben. Nach 18 h wird die Lösung mit 1 M HCl neutralisiert. Die Lösung wird abrotiert und mit Wasser und $CH_2Cl_2$ verdünnt. Die Phasen werden getrennt, die organische Lösung wird getrocknet ($Na_2SO_4$) und einrotiert. Der Rückstand wird säulenchromatographisch getrennt.
Ausbeute: 4,6 g (95 %)
$R_f$ = 0,10 (100:2 = $CH_2Cl_2$ : $CH_3OH$)
MS (DCI, $NH_3$): 408 ($M^+$), 143

**Beispiel XXII**

2-(3,5-Dimethyl-4-hydroxyphenyl)-2-oxo-essigsäure-ethylester

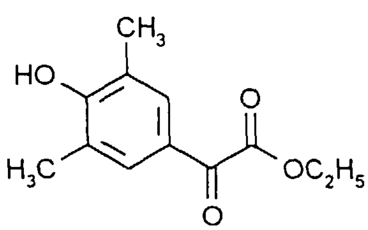

2,6-Dimethylphenol (2 g) wird in Dichlormethan gelöst und mit Ethylchlorooxylat (2,36 g) versetzt. 4,27 g Aluminiumchlorid werden bei 10 bis 20°C portionsweise zugegeben, und die Lösung wird auf RT erwärmt. Nach 18 Stunden wird die Lösung auf Eis gegossen und mit Dichlormethan extrahiert. Die organische Phase wird getrocknet (Natriumsulfat), eingeengt und chromatographiert (Kieselgel 60, Merck, Toluol:Essigester = 4:1).
Ausbeute: 83 %
Fp. = 113°C
$R_f$ = 0,52 (Essigester:Toluol = 1:4)

**Beispiel XXIII**

2-[3,5-Dimethyl-4-(chinolin-2-yl-methoxy)phenyl]-2-oxo-essigsäure-ethylester

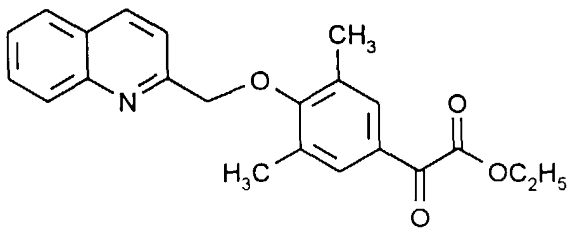

Die Verbindung aus Beispiel Nr. XXII wird analog der Vorschrift von Beispiel Nr. XX umgesetzt.
Ausbeute: 63 %
Fp. = 72°C
$R_f$ = 0,40 (Essigester:Toluol = 1:9)

**Beispiel XXIV**

2-[3,5-Dimethyl-4-(chinolin-2-yl-methoxy)phenyl]-2-hydroxy-2-cycloheptylessigsäureethylester

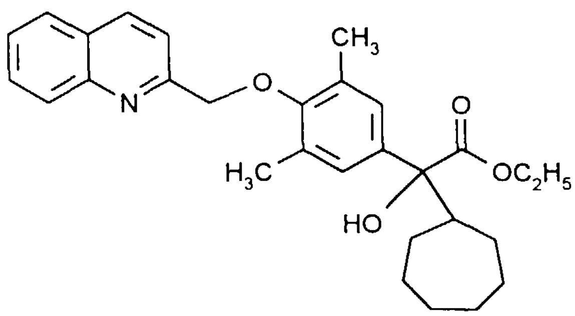

Magnesium (0,4 g) wird in Diethylether vorgelegt und mit 2,94 g Cycloheptylbromid versetzt. Die Reaktionsmischung siedet und wird weiter unter Rückfluß gekocht (1 h). Die Lösung wird auf 0°C gekühlt, und tropfenweise in eine Lösung von 2-[3,5-Dimethyl-4-(chinolin-2-yl-methoxy)phenyl]-2-oxo-essigsäure-ethylester (3 g) in Tetrahydrofuran (20 ml) gegeben. Nach 18 stündigem Nachrühren wird die Lösung in Eiswasser gegossen und mit Essigester extrahiert. Die organische Phase wird getrocknet ($Na_2SO_4$) und chromatographiert (Kieselgel 60, Merck, chlormethan: Essigester:Essigsäure = 100:5:1).
Ausbeute: 600 mg (16 %)
$R_f$ = 0,60 (Dichlormethan:Essigester:Essigsäure = 100:5:1)

**Beispiel XXV**

2-[3,5-Dimethyl-4-(chinolin-2-yl-methoxy)phenyl]-2-hydroxy-2-cycloheptylessigsäure

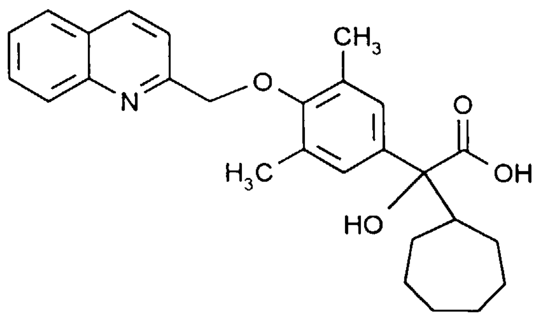

Die Verbindung aus Beispiel XXIV wird analog der Vorschrift von Beispiel XXI umgesetzt.
Ausbeute: 76 %
Fp. = 171°C
$R_f$ = 0,60 (Toluol:Essigester:Essigsäure = 8:2:1)

**Herstellungsbeispiele**

**Beispiel 1 und Beispiel 2**

2-(S)- und 2-(R)-2-{4-[(Chinolin-2-yl)methoxy]phenyl}-2-cyclopentyl-essigsäure-(R)-phenylglycinolamid

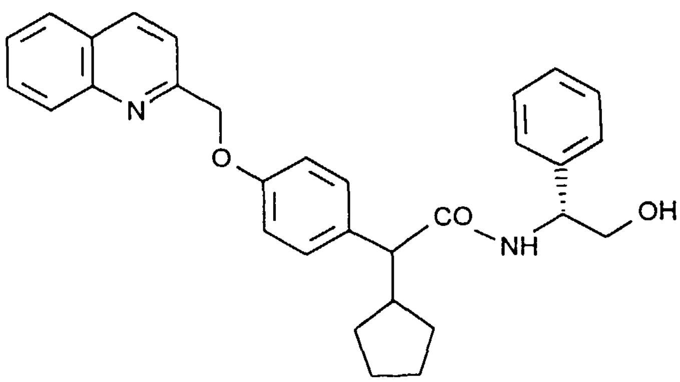

2,5 g (6,9 mmol) racemische 2-{4-[(Chinolin-2-yl)methoxy]phenyl}-2-cyclopentylessigsäure (Synthese: US 4 970 215) werden in 25 ml wasserfreiem N,N-Dimethylformamid gelöst, mit 2,88 ml (20,8 mmol) Triethylamin sowie 604,7 µl (7,6 mmol) Mesylchlorid versetzt und unter Argon als Schutzgas 30 Minuten bei 60°C nachgerührt. Darauf setzt man 1,14 g (8,3 mmol) (R)-Phenylglycinol (käuflich bei Aldrich) und 0,84 g (6,9 mmol) 4-(N,N-Dimethylamino)-pyridin gelöst in 20 ml wasserfreiem N,N-Dimethylformamid zu und rührt unter langsamer Erwärmung auf Raumtemperatur insgesamt 16 h nach. Das Reaktionsgemisch wird mit Essigester und Wasser versetzt und die wäßrige Phase mit 1 M Salzsäure auf einen pH-Wert von ca. 2 gestellt. Die organische Phase wird mehrfach mit verdünnter Salzsäure (pH ≈ 2) extrahiert, mit Wasser gewaschen und dann mehrfach mit 0,1 M wäßriger Natronlauge extrahiert. Wiederum wird die organische Phase mit Wasser gewaschen, anschließend mit wasserfreiem Magnesiumsulfat getrocknet und eingedampft. Zur Auftrennung der Komponenten wird eine Säulenchromatographie durchgeführt (Kieselgel 60, Merck, 40-63 pm, Laufmittel:Petrolether:Essigester von 5:1 bis 1:1).

Beispiel 1:

$R_f$ = 0,12 (Dichlormethan : Essigester = 5:1)
Ausbeute: 1,1 g

Beispiel 2:

Rf = 0,08 (Dichlormethan : Essigester = 5:1)
Ausbeute: 1,0 g

Die absolute Konfiguration der enantiomerenreinen Carbonsäuren (Ausgangsmaterial aus Beispiel 1 und 2) ist bekannt (EP 509 359), sodaß die absolute Konfiguration der Produkte (Beispiel 1 und 2) daraus abgeleitet werden kann. Die 1H-NMR-Spektren der beiden diastereomeren Produkte (200 MHz, d6-DMSO, TMS für Beispiel 1 / 250 MHz, d6-DMSO, TMS für Beispiel 2) weisen im Aromatenbereich signifikante Unterschiede auf:
Die H-Signale des Phenylrestes des Phenylglycinolamids aus Beispiel 1 treten in 2 Gruppen (Integration: 2H und 3H bei ca. 7,1 und 7,3 ppm) auf; im Falle von Beispiel 2 liegt eine Gruppe (Integration: 5H) bei ca. 7,3 ppm. Dieser Befund ist auf viele Derivate diesen Typs übertragbar.

**Beispiel 3 und Beispiel 4**

**2-[3-Pyrrolyl-4-(chinolin-2-yl-methoxy)phenyl)-2-cylcopentylessigsäure (R)-phenylglycinolamid**

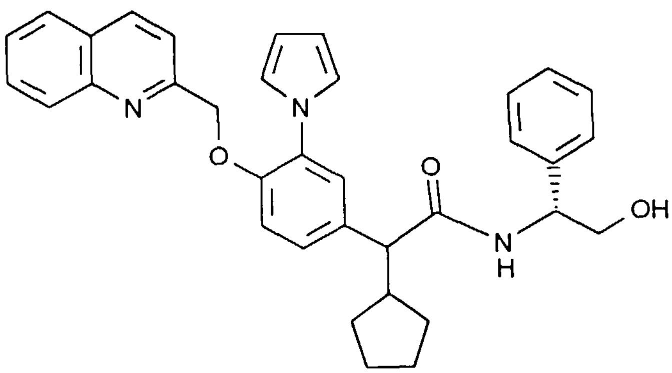

0,445 g der Verbindung aus Beispiel XIV (1,04 mmol), 0,143 g (R)-Phenylglycinol (1,04 mmol) und 0,155 g 1-Hydroxy-1-benzotriazol x $H_2O$ werden in 10 ml $CH_2Cl_2$ gelöst. Die Lösung wird auf 0°C gekühlt und portionsweise mit 0,23 g N'-(3-Dimethylaminopropyl)-N-ethylcarbodiimid Hydrochlorid (1,2 mmol) versetzt. Nach 5 min läßt man 0,21 g Triethylamin (2,08 mmol) dazutropfen, und die Lösung auf 25°C erwärmen. Weitere 15 ml $CH_2Cl_2$ werden dazugegeben und die Lösung wird mit wäßriger $NH_4Cl$- und $NaHCO_3$-Lösung sowie mit Wasser gewaschen. Die Lösung wird getrocknet ($Na_2SO_4$) und einrotiert. Der Rückstand wird säulenchromatographisch getrennt. Man erhält 0,27 g (49% d.Th. eines 1:1 Gemisches der Diastereomeren). Die weitere Auftrennung erfolgt durch präparative HPLC (250 mm Kromasil 100 C-18; 65% $CH_3CN$ in Wasser).

Beispiel 3:

Rf = 0,36 (100:5 = $CH_2Cl_2$: $CH_3OH$)
MS (FAB): 546 $M^+$

Beispiel 4:

Rf= 0,31 (100:5 = $CH_2Cl_2$: $CH_3OH$)
MS (FAB): 546 $M^+$

**Beispiel 5**

2-[2-Fluor-4-(chinolin-2-yl-methoxy)phenyl]-2-cycloheptylessigsäure-(R)-phenylglycinolamid

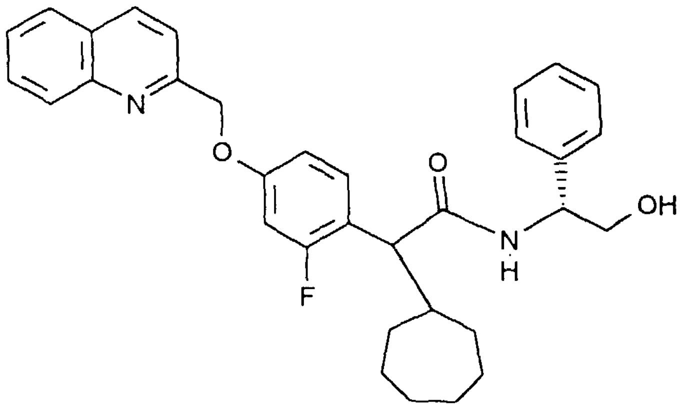

4,6 g der Verbindung aus Beispiel XXI, 1,6 g (R)-Phenylglycinol, und 1,7 g 1-Hydroxy-1-benzotriazol x $H_2O$ werden in 100 ml $CH_2Cl_2$ gelöst. Die Lösung wird auf 0°C gekühlt und 2,5 g N'-(3-Dimethylaminopropyl)-N-ethylcarbodiimid Hydrochlorid werden portionsweise dazugegeben. Nach fünf Minuten werden tropfenweise 2,3 g Triethylamin dazugegeben, und man läßt die Lösung auf 25°C erwärmen. Weiteres $CH_2Cl_2$ wird zugegeben, und die Lösung wird mit $NH_4Cl$- und $NaHCO_3$-Lösung sowie mit Wasser gewaschen. Die Lösung wird mit $Na_2SO_4$ getrocknet und einrotiert. Der Rückstand wird säulenchromatographisch getrennt.
Ausbeute: 4,9 g (82%, 1:1 Diastereomerengemisch)
Rf= 0,77 (10:1 = $CH_2Cl_2$: $CH_3OH$)
Fp. = 167 - 168°C

In Analogie zu den Vorschriften der Beispiele 1 und 2 können die Verbindungen in den Tabellen 1 bis 7 hergestellt werden:

Tabelle 1:

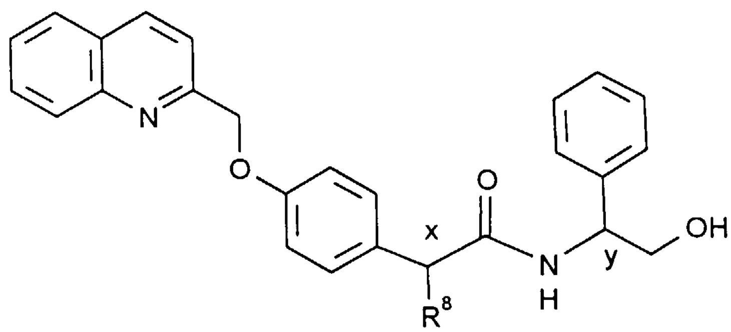

| Bsp.-Nr. | x | y | $R^8$ | $R^f$ (Solvens) | Fp. [°C] | Ausgangs-Verbindung* (Carbonsäure) |
|---|---|---|---|---|---|---|
| 6 | - | R | H | - | >260 | 1) |
| 7 | rac | R | Me | 0,33/0,20 (D) | | 1) |
| 8 | rac | R | Et | 0,28 (E) | | 1) |
| 9 | rac | R | nPr | 0,29 (E) | | 1) |
| 10 | rac | R | iPr | 0,15 (E) | | 1) |
| 11 | rac | R | nBu | 0,51 (D) | | 1) |
| 12 | rac | R | sBu(rac) | 0,45 (E) | | 1) |
| 13 | rac | R | iBu | 0,46 (E) | | 1) |
| 14 | rac | R | E-CH3CH=CHCH2 | 0,31 (E) | | 1) |
| 15 | rac | R | CH3(CH2)4 | 0,31 (E) | | 1) |
| 16 | rac | R | (C2H5)2CH | 0,55 (C) | | 1) |

1) US 4 929 629

| Bsp.-Nr. | x | y | R8 | Rf (Solvens) | Fp. [°C] | Ausgangs-Verbindung* (Carbonsäure) |
|---|---|---|---|---|---|---|
| 17 | rac | R | (CH3)2C=CHCH2 | 0,27 (E) | | 1) |
| 18 | rac | R | (CH3)2C=CH(CH2)2 | 0,49 (E) | | 1) |
| 19 | rac | R | OMe | 0,26/0,13 (D) | | 1) |
| 20 | diaA | S | Me | | 156-159 | 2) |
| 21 | diaB | S | Me | | 144-146 | 2) |
| 22 | S | S | cPent | 0,12 (H) | | 2) |
| 23 | R | S | cPent | 0,08 (H) | | 2) |
| 24 | rac | R | cHex | 0,52/0,59 (C) | | 2) |
| 25 | S | R | cHex | 0,52 (D) | | 2) |
| 26 | R | R | cHex | 0,59 (D) | | 2) |
| 27 | S | R | cHept | | 176 | 2) |
| 28 | R | S | cHept | | 160-162 | 2) |
| 29 | S | S | cHept | | 192-194 | 2) |
| 30 | R | R | cHept | | 191-193 | 2) |
| 31 | rac | R | cOct | 0,14/0,09 (F) | | 3) |
| 32 | S | R | cOct | 0,14 (F) | | 3) |
| 33 | R | R | cOct | 0,09 (F) | | 3) |
| 34 | rac | R | cNon | 0,54/0,44 (E) | | Bsp. VII |
| 35 | S | R | cNon | 0,44 (E) | | Bsp. VII |
| 36 | R | R | cNon | 0,54 (E) | | Bsp. VII |

1) US 4 929 629
2) US 4 970 215
3) US 5 091 392

| Bsp.-Nr. | x | y | R8 | Rf (Solvens) | Fp. [°C] | Ausgangs-Verbindung* (Carbonsäure) |
|---|---|---|---|---|---|---|
| 37 | rac | R | cDec | 0,29/0,39 (E) | | 3) |
| 38 | S | R | cDec | 0,29 (E) | | 3) |
| 39 | R | R | cDec | 0,39 (E) | | 3) |
| 40 | rac | R | cUndec | 0,33/0,44 (E) | | Bsp. Nr. VIII |
| 41 | rac | R | cDodec | 0,20/0,11 (G) | | 3) |
| 42 | rac | R | cPent-CH2 | 0,36 (E) | | 2) |
| 43 | rac | R | cHex-CH2 | 0,69 (C) | | 2) |
| 44 | rac | R | 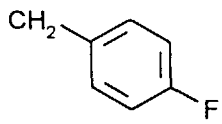 | 0,62 (C) | | |
| 45 | rac | R | rac 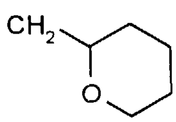 | 0,46 (C) | | |

1) US 4 929 629
2) US 4 970 215
3) US 5 091 392

Tabelle 2:

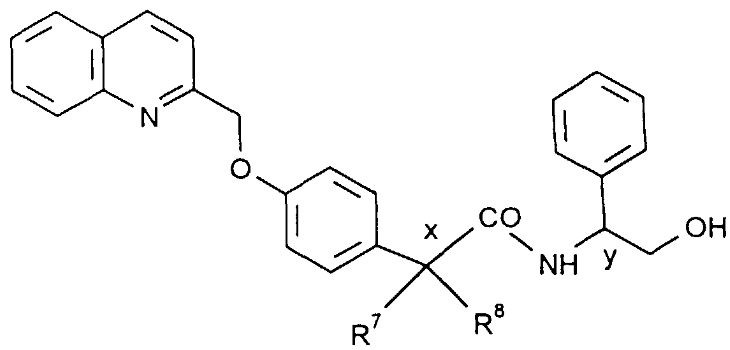

| Bsp.-Nr. | x | y | R7 | R8 | Rf (Solvens) | Fp. [°C] | Ausgangs-Verbindung* (Carbonsäure) |
|---|---|---|---|---|---|---|---|
| 46 | rac | R | OH | Et | 0,55/0,57 (C) | | |
| 47 | rac | R | OH | nBu | 0,39/0,46 (C) | | |
| 48 | rac | R | OH | iBu | 0,31 (E) | | |
| 49 | rac | R | OH | cPent | 0,39 (E) | | 4) |
| 50 | rac | R | OH | cHex | 0,47 (C) | | 4) |
| 51 | dia A | R | OH | cHex | 0,47 (C) | | 4) |
| 52 | dia B | R | OH | cHex | 0,47 (C) | | 4) |
| 53 | dia A | R | OH | cHept | | 215-217 | 5) |

4) EP 414 078
5) US 5 126 354

| Bsp. Nr. | x | y | R7 | R8 | Rf (Solvens) | Fp. [°C] | Ausgangsmaterial (Carbonsäure) |
|---|---|---|---|---|---|---|---|
| 54 | dia B | R | OH | cHept | | 162-164 | 5) |
| 55 | dia A | S | OH | cHept | | 226 | 5) |
| 56 | dia B | S | OH | cHept | | 164-166 | 5) |
| 57 | rac | R | OH | 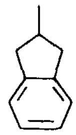 | 0,54 (C) | | 6) |
| 58 | rac | R | OCH3 | cHept | 0,61/0,45 (E) | | |
| 59 | dia A | R | OCH3 | cHept | 0,61 (E) | | |
| 60 | dia B | R | OCH3 | cHept | 0,45 (E) | | |
| 61 | rac | R | F | cHept | 0,49/0,58 (E) | | |

5) US 5 126 354
6) EP 529 450

Tabelle 3:

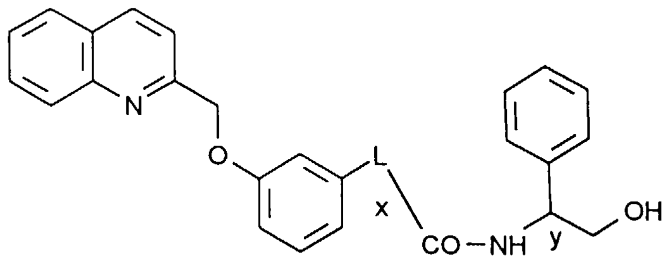

| Bsp. Nr | x | y | L | Rf (Solvens) |
|---|---|---|---|---|
| 62 | / | R | Einfach-Bindung | 0,27 (D) |
| 63 | / | R | -CH2- | 0,42 (C) |
| 64 | rac | R | 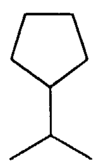 | 0,61 (D) |
| 65 | rac | R | 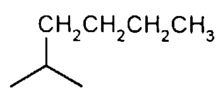 | 0,51 (D) |
| 66 | rac | R | 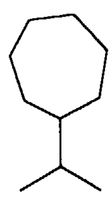 | 0,74 (D) |

| Bsp. Nr. | x | y | L | $R_f$ (Solvens) |
|---|---|---|---|---|
| 67 | rac | R | 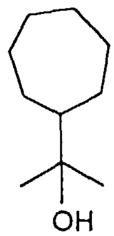 | 0,52 (D) |

Tabelle 4:

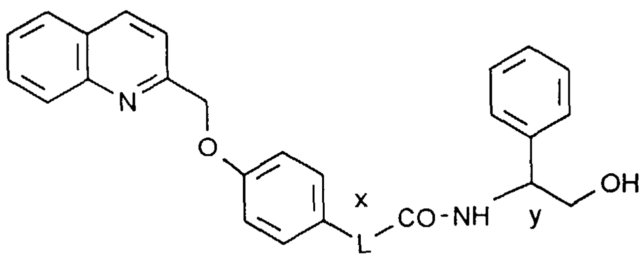

| Bsp. Nr. | x | y | L | $R_f$ (Solvens) | Ausgangsverbindungen (Carbonsäure) |
|---|---|---|---|---|---|
| 68 | / | R | Einfach-Bindung | 0,49 (C) | |
| 69 | / | R | 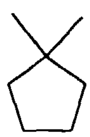 | 0,67 (C) | 7) |

7) EP 414 076

| Bsp. Nr. | x | y | L | $R_f$ (Solvens) | Ausgangsverbindungen (Carbonsäure) |
|---|---|---|---|---|---|
| 70 | / | R | 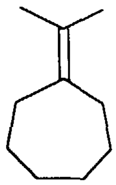 | 0,31 (B) | 7) |
| 71 | / | R | 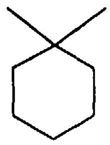 | 0,36 (E) | 7) |

Tabelle 5:

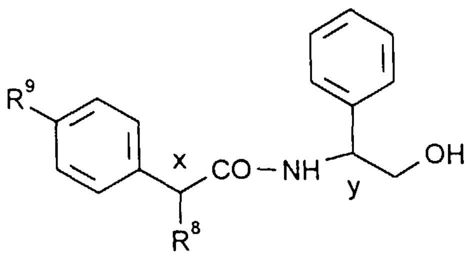

| Bsp. Nr. | x | y | $R^9$ | $R^8$ | Rf (Solvens) | Fp. [°C] |
|---|---|---|---|---|---|---|
| 72 | rac | R | 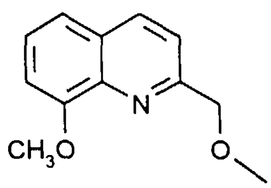 | cPent | | 182-183 |

| Bsp.Nr. | x | y | $R^9$ | $R^8$ | Rf (Solvens) | Fp. [°C] |
|---|---|---|---|---|---|---|
| 73 | rac | R | 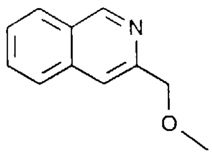 | cPent | 0,50 (A) | |
| 74 | rac | R | 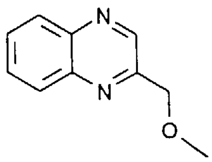 | cPent | | 156-159 |
| 75 | rac | R | 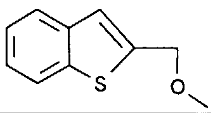 | cPent | | 163-164 |
| 76 | rac | R | 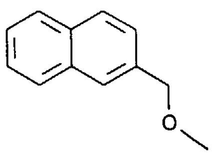 | cPent | | 163 |
| 77 | rac | R | 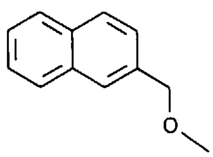 | cHept | 0,62/0,71 (C) | |
| 78 | rac | R | 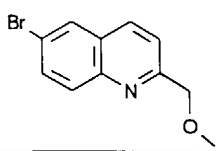 | cPent | | 179-181 |
| 79 | rac | R | 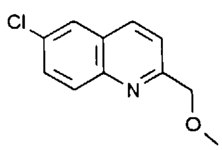 | cPent | | 183-185 |
| 80 | rac | R | 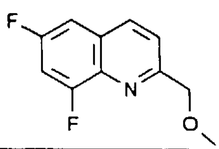 | cPent | | 198-200 |

Tabelle 6:

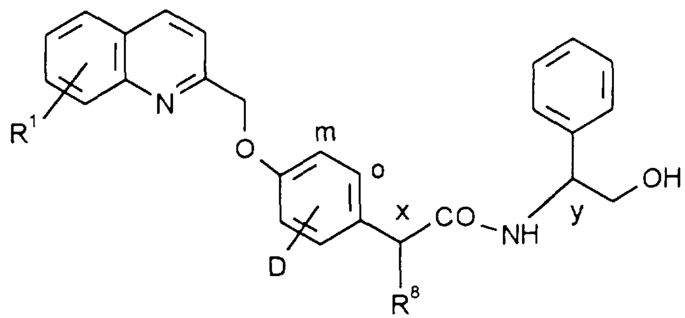

| Bsp.Nr. | x | y | $R^1$ | D | $R^8$ | $R_f$ (Solvens) | Fp.[°C] | MS FAB | Ausgangs-verbindung |
|---|---|---|---|---|---|---|---|---|---|
| 81 | R/S | R | H | m-$OCH_3$ | cHept | 0,27 (E) | | 538 | DE 4 105 551 |
| 82 | R/S | R | H | m-F | cHept | 0,30 (E) | | 526 | DE 4 105 551 |
| 83 | R/S | R | H | m-Cl | cHept | 0,20 (E) | | 543 | DE 4 105 551 |
| 84 | R/S | R | H | (m)-NH-Mes | cPent | 0,20 (E) | | 574 ($M^+$) | Bsp.-Nr. XIII |
| 85 | R/S | R | H | (m)-NHpTos | cPent | 0,32 (E) | | 650 ($M^+$) | Bsp. XV |
| 86 | R/S | R | H | cPr | cPent | 0,46 (E) | 145-153 | 521 | DE 4 105 551 |

| Bsp. Nr. | x | y | $R^1$ | D | $R^8$ | $R_f$ (Solvens) | Fp. [°C] | MS FAB | Ausgangs-verbindung |
|---|---|---|---|---|---|---|---|---|---|
| 87 | R/S | R | H | (m)-i-Bu | cPent | 0,42 (E) | 174-178 | 537 | DE 4 105 551 |
| 88 | R/S | R | H | (m)-Et | cPent | 0,49 (E) | 170-180 | | DE 4 105 551 |
| 89 | R/S | R | H | (m)-Cl | cPent | 0,51 (E) | 175-185 | | DE 4 105 551 |
| 90 | R/S | R | H | (m)-F | cPent | 0,41 (E) | 148-153 | 499 | DE 4 105 551 |
| 91 | R | R | H | (m)-$N_3$ | cPent | 0,72 (C) | 187-189 | | DE 4 105 551 |
| 92 | S | R | H | (m)-$N_3$ | cPent | 0,72 (C) | 165-166 | | DE 4 105 551 |
| 93 | R/S | R | H | (m)-$CH_2CH{=}CH_2$ | cPent | 0,77 (C) | | | DE 4 105 551 |
| 94 | R/S | R | H | (m)-E-$CH{=}CHCH_3$ | cPent | 0,77 (C) | | 521 | DE 4 105 551 |
| 95 | R/S | R | H | (o)-Br | cPent | 0,64 (E) | | | EP 582 908 |
| 96 | R/S | R | H | (o)-nPr | cPent | 0,56 (E) | 157 | 523 | EP 582 908 |
| 97 | R/S | R | H | m-Br | cPent | 0,81 (C) | | | DE 4 105 551 |
| 98 | R/S | R | H | m-$OCH_3$ | cPent | 0,81 (E) | | | DE 4 105 551 |
| 99 | R/S | R | OH | m,m'-$Me_2$ | cHept | 0,29 (E) | | 553 | Bsp.Nr. XXV |

Die Verbindungen folgender Tabelle 7 werden analog der Vorschrift von Beispiel 1 und 2 hergestellt:

**Tabelle 7:**

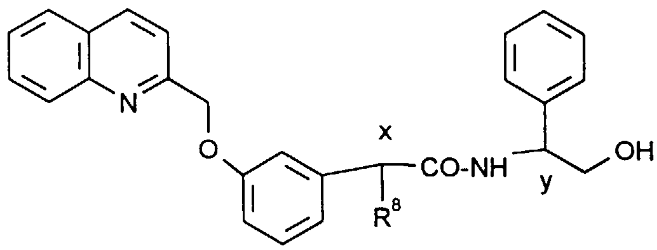

| Bsp. Nr. | x | y | $R^8$ | Fp. [°C] |
|---|---|---|---|---|
| 100 | rac | S | nBu | 138 |
| 101 | rac | S | cPent | 134-138 |
| 102 | rac | S | cHex | 145-150 |
| 103 | rac | S | cHept | 127-129 |

Die Verbindungen folgender Tabelle 8 werden analog der Vorschrift von Beispiel 1 und 2 hergestellt:

**Tabelle 8:**

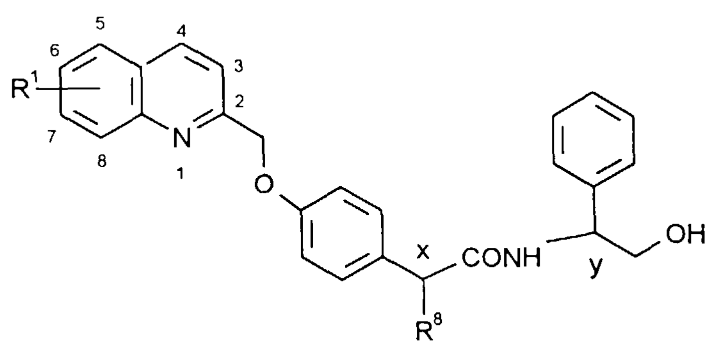

| Bsp. Nr. | x | y | $R^1$ | $R^8$ | Fp. [°C] |
|---|---|---|---|---|---|
| 104 | rac | S | 6-F | cHept | 161-167 |
| 105 | rac | S | 6-Br | cPent | 172-176 |
| 106 | rac | S | 6-F | cPent | 189-193 |
| 107 | rac | S | 7-Cl | cPent | 202 |

Die Verbindungen folgender Tabelle 9 werden analog der Vorschrift von Beispiel 1 und 2 hergestellt:

**Tabelle 9:**

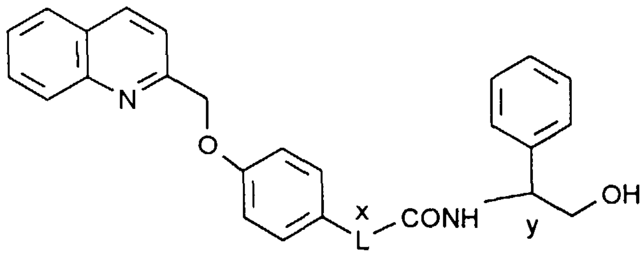

| Bsp. Nr. | x | y | 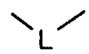 | Fp. [°C] |
|---|---|---|---|---|
| 108 | | S | 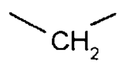 | >260 |
| 109 | | S | 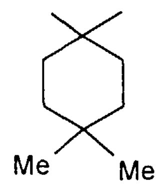 | 146-147 |

| Bsp. Nr. | x | y | 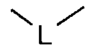 | Fp. [°C] |
|---|---|---|---|---|
| 110 | rac | S | 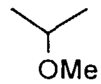 | 156-161 |
| 111 | rac | S | 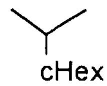 | 167-169 |
| 112 | rac | S | 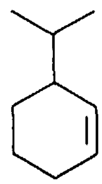 | 165-168 |
| 113 | | S | 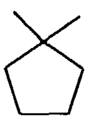 | 159-161 |
| 114 | | S | 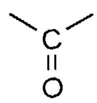 | 174-176 |

Tabelle 10:

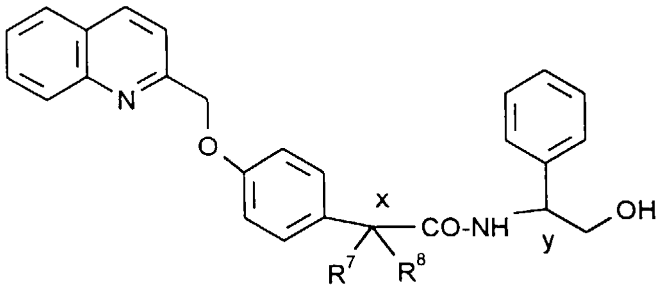

| Bsp. Nr. | x | y | R7 | R8 | Fp. [°C] |
|---|---|---|---|---|---|
| 115 | rac | S | H | (CH3)2C=CHCH2 | 150-153 |
| 116 | rac | S | H | cPrCH2 | 162-164 |
| 117 | rac | S | H | Et | 180-182 |
| 118 | rac | S | H | CH2SiMe3 | 130-133 |
| 119 | rac | S | H | nBu | 140-143 |
| 120 | rac | S | H | cHexCH2 | 177-181 |

Die Verbindungen folgender Tabelle 11 werden analog der Vorschrift von Beispiel 1 und 2 hergestellt:

**Tabelle 11:**

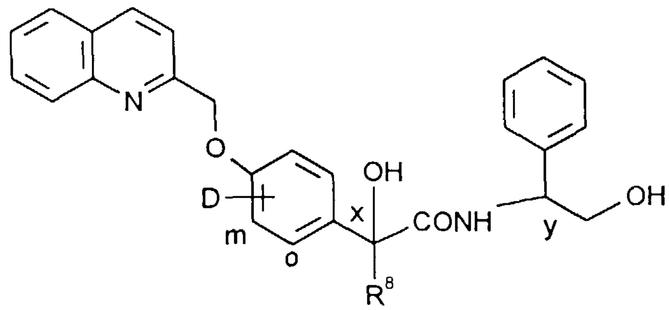

| Bsp. Nr. | x | y | D | $R^8$ | Fp. [°C] |
|---|---|---|---|---|---|
| 121 | dia A | S | H | cPent | 168 |
| 122 | dia B | S | H | cPent | 153 |
| 123 | rac | S | H | cPent | 158-162 |
| 124 | dia B | S | H | cHex | 171 |
| 125 | dia A | S | H | cHex | 223 |
| 126 | dia A | S | m-iBu | cPent | 151 |
| 127 | dia A | S | m-iBu | cHept | 183 |
| 128 | dia B | S | m-iBu | cHept | 183 |

## Patentansprüche

1. Verwendung von bicyclisch substituierten Oxy-phenyl-(phenyl) glycinolamiden der allgemeinen Formel (I)

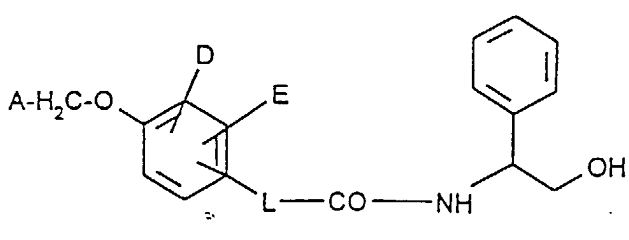

(I)

in welcher

A für Naphthyl
oder für einen Rest der Formel

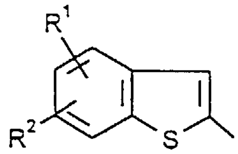, 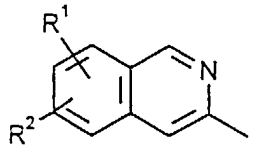, 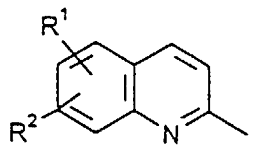,

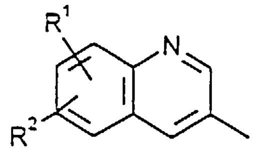, 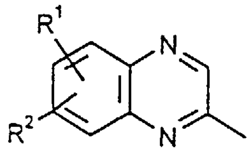 oder 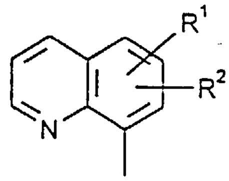

steht,
worin

$R^1$ und $R^2$ gleich oder verschieden sind und Wasserstoff, Halogen, Hydroxy oder geradkettiges oder verzweigtes Alkoxy mit bis zu 4 Kohlenstoffatomen bedeuten,

D und E gleich oder verschieden sind und
für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen, einen 5- bis 6-gliedrigen ungesättigten oder gesättigten Heterocyclus mit bis zu 3 Heteroatomen aus der Reihe S, N und/oder O stehen,
oder
für einen Rest der Formel $-NR^3SO^4$ oder $-NR^5SO_2-R^6$ stehen,
worin

$R^3$, $R^4$ und $R^5$ gleich oderverschieden sind und Wasserstoff oder geradkettiges oder verzweigtes Alkyl mit bis zu 4 Kohlenstoffatomen oder Phenyl bedeuten,

$R^6$ geradkettiges oder verzweigtes Alkyl mit bis zu 4 Kohlenstoffatomen, Benzyl oder Phenyl bedeutet, das gegebenenfalls durch geradkettiges oder verzweigtes Alkyl, mit bis zu 4 Kohlenstoffatomen substituiert ist,

L für eine Bindung steht,
oder
für einen Rest der Formel

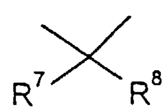

steht,
worin

$R^7$ Wasserstoff, Hydroxy, Methoxy oder Halogen bedeutet,

$R^8$ Wasserstoff, Hydroxy, Halogen, geradkettiges oder verzweigtes Alkenyl oder Alkoxy mit jeweils bis zu 8 Kohlenstoffatomen oder Cycloalkyl oder Cycloalkenyl mit jeweils 3 bis 14 Kohlenstoffatomen bedeutet, oder
geradkettiges oder verzweigtes Alkyl mit bis zu 8 Kohlenstoffatomen bedeutet, das gegebenenfalls durch Cycloalkyl mit 3 bis 8 Kohlenstoffatomen, Phenyl oder Tetrahydropyranyl substituiert ist, welche ihrerseits durch Halogen substituiert sein können,

oder einen Rest der Formel
$-CH_2SiR^9R^{10}R^{11}$ oder einen Indanylrest bedeutet,
worin

$R^9$, $R^{10}$ und $R^{11}$ gleich oder verschieden sind und geradkettiges oder verzweigtes Alkyl mit bis zu 4 Kohlenstoffatomen bedeuten

oder

$R^7$ und $R^8$ gemeinsam mit dem Kohlenstoffatom einen gesättigten carbocyclischen Ring mit 5 bis 7 Kohlenstoffatomen bilden, der gegebenenfalls bis zu 2-fach gleich oder verschieden durch geradkettiges oder verzweigtes Alkyl mit bis zu 4 Kohlenstoffatomen substituiert ist,

oder

$R^7$ und $R^8$ gemeinsam einen Rest der Formel = O oder einen Doppelbindungs-Rest der Formel

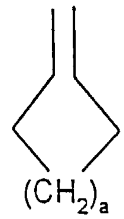

bilden,
worin

a eine Zahl 2, 3, 4, 5 oder 6 bedeutet,

und deren Salze,
zur Herstellung von Arzneimitteln zur Senkung von ApoB-100-assoziirten Lipoproteinen.

**2.** Verwendung von bicyclisch substituierten Oxy-phenyl-(phenyl) glycinolamiden nach Anspruch 1 in welchen

A für Naphthyl oder
für einen Rest der Formel

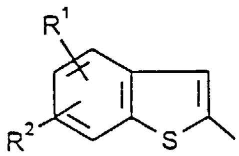,
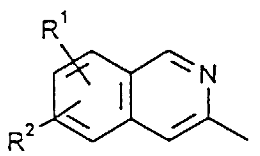,
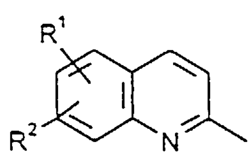,

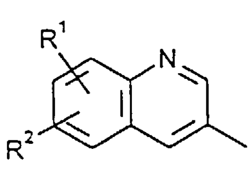,
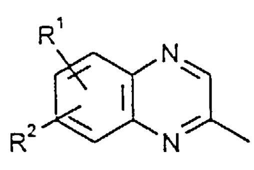
oder
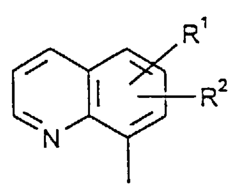

steht,
worin

$R^1$ und $R^2$ gleich oder verschieden sind und Wasserstoff, Fluor, Chlor, Brom, Hydroxy oder geradkettiges oder verzweigtes Alkoxy mit bis zu 3 Kohlenstoffatomen bedeuten,

D und E gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 5 Kohlenstoffatomen, Pyrryl oder Imidazolyl stehen,
oder
für einen Rest der Formel $-NR^3R^4$ oder-$NR^5-SO_2-R^6$ stehen,
worin

$R^3$, $R^4$ und $R^5$ gleich oder verschieden sind und Wasserstoff, geradkettiges oder verzweigtes Alkyl mit bis zu 3 Kohlenstoffatomen oder Phenyl bedeuten,

$R^6$ geradkettiges oder verzweigtes Alkyl mit bis zu 3 Kohlenstoffatomen, Benzyl oder Phenyl bedeutet, das gegebenenfalls durch geradkettiges oder verzweigtes Alkyl mit bis zu 3 Kohlenstoffatomen substituiert ist,

L für eine Bindung steht,
oder
für einen Rest der Formel

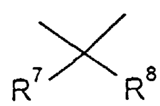

steht, worin

$R^7$ Wasserstoff, Hydroxy, Methoxy, Fluor, Chlor oder Brom bedeutet,

$R^8$ Wasserstoff, Hydroxy, Halogen, geradkettiges oder verzweigtes Alkenyl oder Alkoxy mit jeweils bis zu 7 Kohlenstoffatomen, Cyclopentyl, Cyclohexyl, Cycloheptyl, Cyclooctyl, Cyclononyl, Cyclodecyl, Cycloundecyl, Cyclo-dodecyl oder Cyclohexenyl bedeutet, oder
geradkettiges oder verzweigtes Alkyl mit bis zu 7 Kohlenstoffatomen bedeutet, das gegebenenfalls durch Cyclopentyl, Cyclohexyl, Cycloheptyl, Cyclooctyl, Phenyl oder Tetrahydropyranyl substituiert ist, welche ihrerseits durch Fluor, Chlor oder Brom substituiert sein können,
oder einen Rest der Formel $CH_2-SiR^9R^{10}R^{11}$ oder einen Indanylrest bedeutet,
worin
$R^9$, $R^{10}$ und $R^{11}$ gleich oder verschieden sind und geradkettiges oder verzweigtes Alkyl mit bis zu 3 Kohlenstoffatomen bedeuten

oder

$R^{13}$ und $R^{14}$ gemeinsam mit dem Kohlenstoffatom einen Cyclopentyl-, Cyclohexyl- oder Cycloheptylring bilden, die gegebenenfalls bis zu 2-fach durch Methyl substituiert sind

oder

$R^7$ und $R^8$ gemeinsam einen Rest der Formel = 0 oder einen Doppelbindungs-Rest der Formel

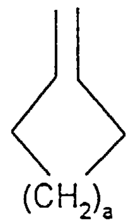

bilden, worin

a eine Zahl 2,3 oder 4 bedeutet,

und deren Salze,
zur Herstellung von Arzneimitteln zur Senkung von ApoB-100-assoziirten Lipoproteinen.

**3.** Verwendung von bicyclisch substituierten Oxy-phenyl-(phenyl) glycinolamiden nach Anspruch 1, in welchen

A für Naphtyl
oder
für einen Rest der Formel

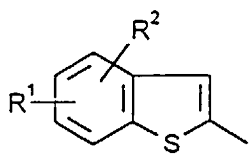
,
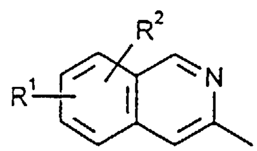
,
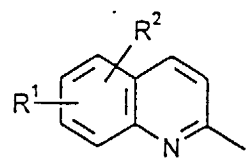
,

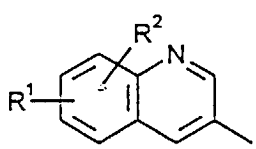
,
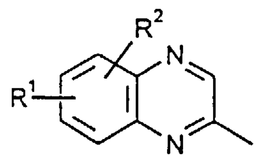
oder
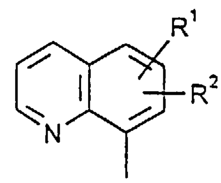

steht,
worin

$R^1$ und $R^2$ gleich oder verschieden sind und Wasserstoff, Fluor, Chlor, Brom, Hydroxy oder Methoxy bedeuten,

D und E gleich oder verschieden sind und
für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 5 Kohlenstoffatomen, Pyrryl oder Imidazolyl stehen,
oder
für einen Rest der Formel $-NR^5-SO_2-R^6$ stehen,
worin

$R^5$ Wasserstoff oder Methyl bedeutet,

$R^6$ geradkettiges oder verzweigtes Alkyl mit bis zu 3 Kohlenstoffatomen, Benzyl oder Phenyl bedeutet, das gegebenenfalls durch Methyl oder Ethyl substituiert ist,

L für eine Bindung steht,
oder
für einen Rest der Formel

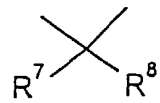

steht,
worin

$R^7$ Wasserstoff, Hydroxy, Fluor oder Methoxy bedeutet,

$R^8$ Wasserstoff, Hydroxy, geradkettiges oder verzweigtes Alkenyl oder Alkoxy mit jeweils bis zu 6 Kohlenstoffatomen, Cyclopentyl, Cyclohexyl, Cycloheptyl, Cyclooctyl, Cyclo-nonyl, Cyclodecyl, Cycloundecyl, Cyclododecyl oder Cyclohexenyl bedeutet, oder
geradkettiges oder verzweigtes Alkyl mit bis zu 6 Kohlenstoffatomen bedeutet, das gegebenenfalls durch Cyclopentyl, Cyclohexyl, Cycloheptyl, Phenyl oder Tetrahydropyranyl substituiert ist, welche ihrerseits durch Fluor, Chlor oder Brom substituiert sein können,
oder einen Rest der Formel $CH_2Si(R^9R^{10}R^{11})$ oder einen Indonylrest bedeutet,
worin

$R^9$, $R^{10}$ und $R^{11}$ Methyl bedeuten

oder

$R^7$ und $R^8$ gemeinsam mit dem Kohlenstoffatom einen Cyclopentyl-, Cyclohexyl- oder Cycloheptylring bilden, die gegebenenfalls bis zu 2-fach durch Methyl substituiert sind

oder

$R^7$ und $R^8$ gemeinsam einen Rest der Formel = 0 oder einen Doppelbindungs-Rest der Formel

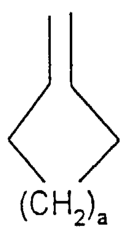

bedeuten,
worin

a eine Zahl 2, 3 oder 4 bedeutet,

und deren Salze,
zur Herstellung von Arzneimitteln zur Senkung von ApoB-100-assoziirten Lipoproteinen.

**4.** Verwendung der ApoB-100-assoziierte Lipoproteine senkenden bicyclisch substituierten Oxy-phenyl-(phenyl)glycinolamide gemäß einem der Ansprüche 1 bis 3 zur Herstellung von Arzneimitteln, zur Senkung des VLDL-Plasmaspiegels.

**5.** Verwendung der ApoB-100-assoziierte Lipoproteine senkenden bicyclisch substituierten Oxy-phenyl-(phenyl)glycinolamide gemäß einem der Ansprüche 1 bis 4 zur Herstellung von Arzneimitteln zur Senkung der Plasmaspiegel von Triglyceriden.

**6.** Verwendung der ApoB-100-assoziierte Lipoproteine senkenden bicyclisch substituierten Oxy-phenyl-(phenyl)glycinolamide gemäß einem der Ansprüche 1 bis 5 zur Herstellung von Arzneimitteln zur Senkung des Plasmaspiegels von Cholesterol.

**7.** Verwendung der ApoB-100-assoziierte Lipoproteine senkenden bicyclisch substituierten Oxy-phenyl-(phenyl)glycinolamide gemäß einem der Ansprüche 1 bis 6, wobei es sich um Arzneimittel zur Behandlung von Erkrankungen, die mit erhöhten Plasmaspiegeln von VLDL, ApoB-100, LDL, Triglyceriden oder Cholesterin assoziiert sind, handelt.

**8.** Verwendung von ApoB-100-assoziierte Lipoproteine senkenden bicyclisch substituierten Oxyphenyl-(phenyl)glycinolamiden gemäß einem der Ansprüche 1 bis 3 zur Herstellung von Arzneimitteln zur Behandlung von Adipositas.

**Claims**

**1.** Use of oxy-phenyl-(phenyl)glycinolamides with bicyclic substituents, of the general formula (I)

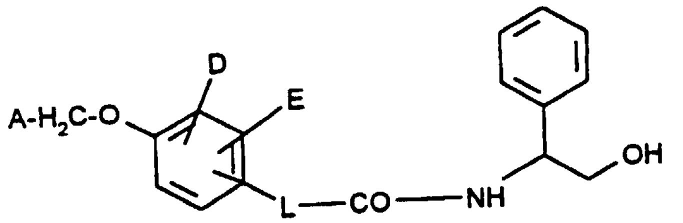

(I)

in which

A represents naphthyl,
or represents a radical of the formula

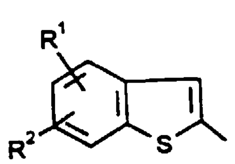 ,
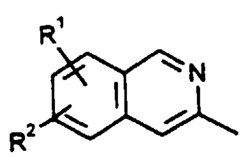 ,
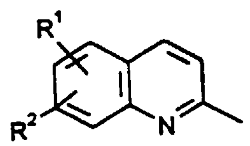 ,

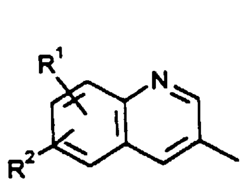 ,
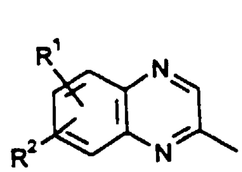 or
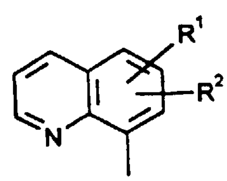

wherein

$R^1$ and $R^2$ are identical or different and denote hydrogen, halogen, hydroxyl or straight-chain or branched alkoxy having up to 4 carbon atoms,

D and E are identical or different and represent hydrogen, cycloalkyl having 3 to 8 carbon atoms, azido, hydroxyl, halogen, straight-chain or branched alkyl, alkoxy or alkenyl having in each case up to 6 carbon atoms, or a 5- to 6-membered unsaturated or saturated heterocyclic radical having up to 3 hetero atoms from the series consisting of S, N and/or O,

or

represent a radical of the formula $-NR^3R^4$ or $-NR^5SO_2-R^6$,

wherein

$R^3$, $R^4$ and $R^5$ are identical or different and denote hydrogen or straight-chain or branched alkyl having up to 4 carbon atoms or phenyl,

$R^6$ denotes straight-chain or branched alkyl having up to 4 carbon atoms, benzyl or phenyl, which is optionally substituted by straight-chain or branched alkyl having up to 4 carbon atoms,

L represents a bond,

or

represents a radical of the formula

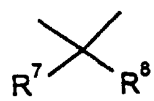

wherein

$R^7$ denotes hydrogen, hydroxyl, methoxy or halogen,

$R^8$ denotes hydrogen, hydroxyl, halogen, straight-chain or branched alkenyl or alkoxy having in each case up to 8 carbon atoms or cycloalkyl or cycloalkenyl having in each case 3 to 14 carbon atoms, or

denotes straight-chain or branched alkyl having up to 8 carbon atoms, which is optionally substituted by cycloalkyl having 3 to 8 carbon atoms, phenyl or tetrahydropyranyl, which in their turn can be substituted by halogen,

or denotes a radical of the formula

$-CH_2SiR^9R^{10}R^{11}$ or an indanyl radical,

wherein

$R^9$, $R^{10}$ and $R^{11}$ are identical or different and denote straight-chain or branched alkyl having up to 4 carbon atoms

or

$R^7$ and $R^8$ together with the carbon atom form a saturated carbocyclic ring having 5 to 7 carbon atoms, which is optionally substituted up to twice in an identical or different manner by straight-chain or branched alkyl having up to 4 carbon atoms,

or

$R^7$ and $R^8$ together form a radical of the formula = O or a double bond radical of the formula

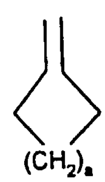

wherein

a denotes the number 2, 3, 4, 5 or 6,

and salts thereof,
for the preparation of medicaments for lowering ApoB-100-associated lipoproteins.

**2.** Use of oxy-phenyl-(phenyl)glycinolamides with bicyclic substituents, according to Claim 1, in which

A represents naphthyl or
represents a radical of the formula

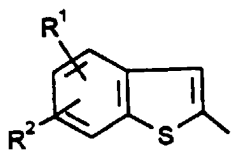 , 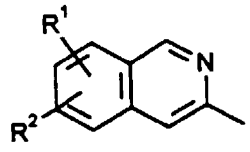 , 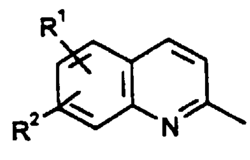 ,

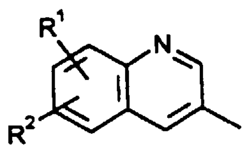 , 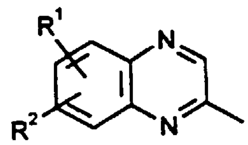 or 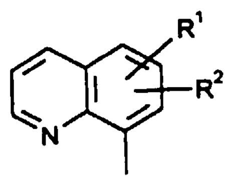

wherein

$R^1$ and $R^2$ are identical or different and denote hydrogen, fluorine, chlorine, bromine, hydroxyl or straight-chain or branched alkoxy having up to 3 carbon atoms,

D and E are identical or different and
represent hydrogen, cyclopropyl, cyclopentyl, cyclohexyl, azido, hydroxyl, fluorine, chlorine, bromine, straight-chain or branched alkyl, alkoxy or alkenyl having in each case up to 5 carbon atoms, pyrryl or imidazolyl,
or
represent a radical of the formula $-NR^3R^4$ or $-NR^5-SO_2-R^6$,
wherein

$R^3$, $R^4$ and $R^5$ are identical or different and denote hydrogen, straight-chain or branched alkyl having up to 3 carbon atoms or phenyl,

$R^6$ denotes straight-chain or branched alkyl having up to 3 carbon atoms, benzyl or phenyl, which is optionally substituted by straight-chain or branched alkyl having up to 3 carbon atoms,

L represents a bond,
or
represents a radical of the formula

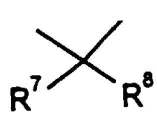

wherein

| | |
|---|---|
| $R^7$ | denotes hydrogen, hydroxyl, methoxy, fluorine, chlorine or bromine, |
| $R^8$ | denotes hydrogen, hydroxyl, halogen, straight-chain or branched alkenyl or alkoxy having in each case up to 7 carbon atoms, cyclopentyl, cyclohexyl, cycloheptyl, cyclooctyl, cyclononyl, cyclodecyl, cycloundecyl, cyclododecyl or cyclohexenyl, or denotes straight-chain or branched alkyl having up to 7 carbon atoms, which is optionally substituted by cyclopentyl, cyclohexyl, cycloheptyl, cyclooctyl, phenyl or tetrahydropyranyl, which in their turn can be substituted by fluorine, chlorine or bromine,<br>or denotes a radical of the formula $CH_2$-$SiR^9R^{10}R^{11}$ or an indanyl radical,<br>wherein<br>$R^9$, $R^{10}$ and $R^{11}$ are identical or different and denote straight-chain or branched alkyl having up to 3 carbon atoms, |

or

| | |
|---|---|
| $R^{13}$ and $R^{14}$ | together with the carbon atom form a cyclopentyl, cyclohexyl or cycloheptyl ring, which are optionally substituted up to twice by methyl, |

or

| | |
|---|---|
| $R^7$ and $R^8$ | together form a radical of the formula = O or a double bond radical of the formula |

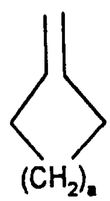

wherein

a denotes the number 2, 3 or 4,

and salts thereof,
for the preparation of medicaments for lowering ApoB-100-associated lipoproteins.

**3.** Use of oxy-phenyl(phenyl)glycinolamides with bicyclic substituents, according to Claim 1, in which

A represents naphthyl
or
represents a radical of the formula

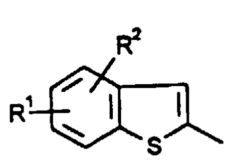 , 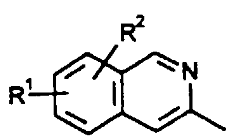 , 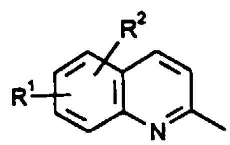 ,

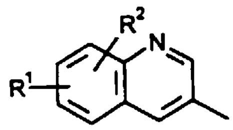,
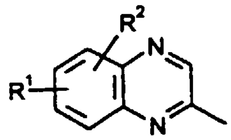
or
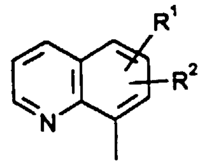

wherein

$R^1$ and $R^2$ are identical or different and denote hydrogen, fluorine, chlorine, bromine, hydroxyl or methoxy,

D and E are identical or different and represent hydrogen, cyclopropyl, cyclopentyl, cyclohexyl, azido, hydroxyl, fluorine, chlorine, bromine, straight-chain or branched alkyl, alkoxy or alkenyl having in each case up to 5 carbon atoms, pyrryl or imidazolyl,
or
represent a radical of the formula $-NR^5-SO_2-R^6$,
wherein

$R^5$ denotes hydrogen or methyl,

$R^6$ denotes straight-chain or branched alkyl having up to 3 carbon atoms, benzyl or phenyl, which is optionally substituted by methyl or ethyl,

L represents a bond,
or
represents a radical of the formula

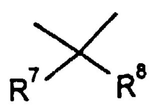

wherein

$R^7$ denotes hydrogen, hydroxyl, fluorine or methoxy,

$R^8$ denotes hydrogen, hydroxyl, straight-chain or branched alkenyl or alkoxy having in each case up to 6 carbon atoms, cyclopentyl, cyclohexyl, cycloheptyl, cyclooctyl, cyclononyl, cyclodecyl, cycloundecyl, cyclododecyl or cyclohexenyl, or denotes straight-chain or branched alkyl having up to 6 carbon atoms, which is optionally substituted by cyclopentyl, cyclohexyl, cycloheptyl, phenyl or tetrahydropyranyl, which in their turn can be substituted by fluorine, chlorine or bromine,
or denotes a radical of the formula $CH_2Si(R^9R^{10}R^{11})$ or an indanyl radical,
wherein
$R^9$, $R^{10}$ and $R^{11}$ denote methyl,

or

$R^7$ and $R^8$ together with the carbon atom form a cyclopentyl, cyclohexyl or cycloheptyl ring, which are optionally substituted up to twice by methyl,

or

$R^7$ and $R^8$ together denote a radical of the formula = O or a double bond radical of the formula

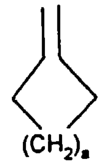

wherein

a denotes the number 2, 3 or 4,

and salts thereof,
for the preparation of medicaments for lowering ApoB-100-associated lipoproteins.

4. Use of oxy-phenyl-(phenyl)glycinolamides with bicyclic substituents, which lower ApoB-100-associated lipoproteins, according to one of Claims 1 to 3, for the preparation of medicaments for lowering the VLDL plasma level.

5. Use of oxy-phenyl-(phenyl)glycinolamides with bicyclic substituents, which lower ApoB-100-associated lipoproteins, according to one of Claims 1 to 4, for the preparation of medicaments for lowering the plasma level of triglycerides.

6. Use of oxy-phenyl-(phenyl)glycinolamides with bicyclic substituents, which lower ApoB-100-associated lipoproteins, according to one of Claims 1 to 5, for the preparation of medicaments for lowering the plasma level of cholesterol.

7. Use of oxy-phenyl-(phenyl)glycinolamides with bicyclic substituents, which lower ApoB-100-associated lipoproteins, according to one of Claims 1 to 6, where the medicaments are for treating disorders associated with increased plasma levels of VLDL, ApoB-100, LDL, triglycerides or cholesterol.

8. Use of oxyphenyl-(phenyl)glycinolamides with bicyclic substituents, which lower ApoB-100-associated lipoproteins, according to one of Claims 1 to 3, for the preparation of medicaments for treating obesity.

## Revendications

1. Utilisation d'oxy-phényl-(phényl) -glycinolamides à substituant bicyclique, de formule générale (I)

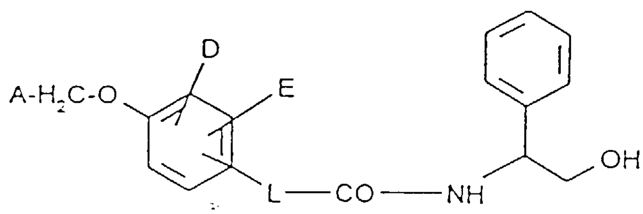

(I)

dans laquelle

A est un reste naphtyle
ou l'un des restes de formule

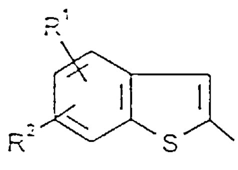
,
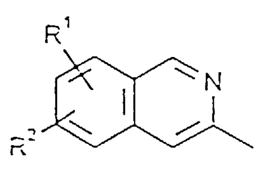
,
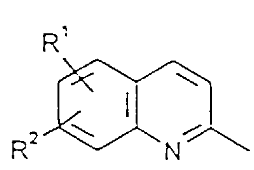
,

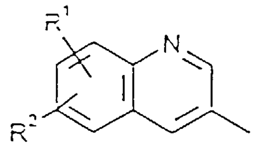 , 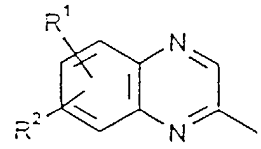 ou 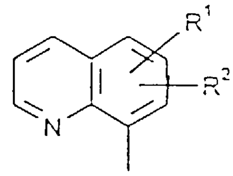

dans laquelle

$R^1$ et $R^2$ sont identiques ou différents et représentent l'hydrogène, un halogène, un groupe hydroxy ou un groupe alkoxy linéaire ou ramifié ayant jusqu'à 4 atomes de carbone,

D et E sont identiques ou différents et représentent l'hydrogène, un groupe cycloalkyle ayant 3 à 8 atomes de carbone, azido, hydroxy, un halogène, un groupe linéaire ou ramifié alkyle, alkoxy ou alcényle ayant dans chaque cas jusqu'à 6 atomes de carbone, un hétérocycle pentagonal ou hexagonal non saturé ou saturé ayant jusqu'à 3 hétéroatomes de la série S, N et/ou O,
ou bien
un reste de formule $-NR^3R^4$ ou $-NR^5SO_2-R^6$
dans laquelle

$R^3$, $R^4$ et $R^5$ sont identiques ou différents et représentent l'hydrogène ou un groupe alkyle linéaire ou ramifié ayant jusqu'à 4 atomes de carbone ou le groupe phényle,
$R^6$ est un groupe alkyle linéaire ou ramifié ayant jusqu'à 4 atomes de carbone, benzyle ou phényle, qui est substitué le cas échéant par un radical alkyle linéaire ou ramifié ayant jusqu'à 4 atomes de carbone,

L représente une liaison,
ou bien
un reste de formule

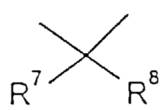

dans laquelle

$R^7$ est l'hydrogène, un groupe hydroxy, méthoxy ou un halogène,
$R^8$ est l'hydrogène, un groupe hydroxy, un halogène, un groupe alcényle ou alkoxy linéaire ou ramifié ayant dans chaque cas jusqu'à 8 atomes de carbone ou un groupe cycloalkyle ou cycloalcényle ayant chacun 3 à 14 atomes de carbone, ou bien
un groupe alkyle linéaire ou ramifié ayant jusqu'à 8 atomes de carbone, qui est substitué le cas échéant par un radical cycloalkyle ayant 3 à 8 atomes de carbone, phényle ou tétrahydropyrannyle, ces radicaux pouvant eux-mêmes être substitués par un halogène,
ou un reste de formule $-CH_2SiR^9R^{10}R^{11}$ ou un reste indanyle,
où

$R^9$, $R^{10}$ et $R^{11}$ sont identiques ou différents et représentent un groupe alkyle linéaire ou ramifié ayant jusqu'à 4 atomes de carbone,

ou bien
$R^7$ et $R^8$ forment conjointement avec l'atome de carbone un noyau carbocyclique saturé ayant 5 à 7 atomes de carbone, qui est substitué, le cas échéant, jusqu'à deux fois identiques ou différentes par un radical alkyle linéaire ou ramifié ayant jusqu'à 4 atomes de carbone,

ou bien

$R^7$ et $R^8$ forment ensemble un reste de formule =O ou un reste à double liaison de formule

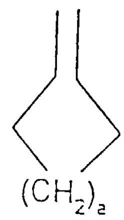

dans laquelle

a représente le nombre 2, 3, 4, 5 ou 6

et de leurs sels,
pour la préparation de médicaments destinés à abaisser le taux de lipoprotéines asociées à l'ApoB-100.

**2.** Utilisation d'oxy-phényl-(phényl)-glycinolamides à substituant bicyclique suivant la revendication 1, dans lesquels

A est un reste naphtyle
ou l'un des restes de formule

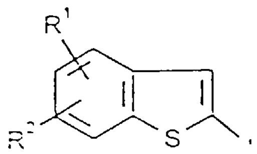 , 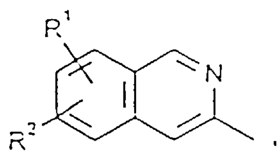 , 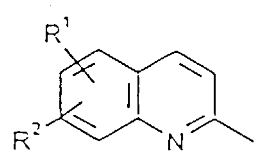 ,

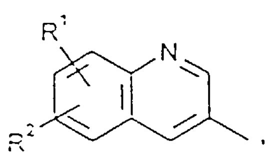 , 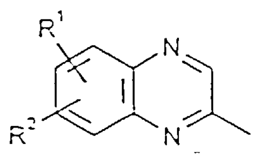 ou 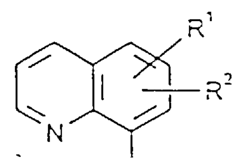

dans laquelle

$R^1$ et $R^2$ sont identiques ou différents et représentent l'hydrogène, le fluor, le chlore, le brome, un groupe hydroxy ou un groupe alkoxy linéaire ou ramifié ayant jusqu'à 3 atomes de carbone,

D et E sont identiques ou différents et représentent l'hydrogène, un groupe cyclopropyle, cyclopentyle, cyclohexyle, azido, hydroxy, le fluor, le chlore, le brome, un groupe alkyle, alkoxy ou alcényle linéaire ou ramifié ayant dans chaque cas jusqu'à 5 atomes de carbone, le groupe pyrryle ou le groupe imidazolyle, ou bien
un reste de formule $-NR^3R^4$ ou $-NR^5SO_2-R^6$
dans laquelle

$R^3$, $R^4$ et $R^5$ sont identiques ou différents et représentent l'hydrogène, un groupe alkyle linéaire ou ramifié ayant jusqu'à 3 atomes de carbone ou le groupe phényle,
$R^6$ est un groupe alkyle linéaire ou ramifié ayant jusqu'à 3 atomes de carbone, un groupe benzyle ou phényle, qui est substitué le cas échéant par un radical alkyle linéaire ou ramifié ayant jusqu'à 3 atomes de carbone,

L représente une liaison,
ou bien

un reste de formule

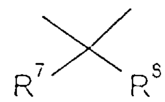

dans laquelle

$R^7$ est l'hydrogène, un groupe hydroxy, méthoxy, le fluor, le chlore ou le brome,

$R^8$ est l'hydrogène, un groupe hydroxy, un halogène, un groupe alcényle ou alkoxy linéaire ou ramifié ayant dans chaque cas jusqu'à 7 atomes de carbone, un groupe cyclopentyle, cyclohexyle, cycloheptyle, cyclo-octyle, cyclononyle, cyclodécyle, cyclo-undécyle, cyclododécyle ou cyclohexényle, ou bien un groupe alkyle linéaire ou ramifié ayant jusqu'à 7 atomes de carbone, qui est substitué le cas échéant par un radical cyclopentyle, cyclohexyle, cycloheptyle, cyclo-octyle, phényle ou tétrahydropyrannyle, ces radicaux pouvant être substitués quant à eux par du fluor, du chlore ou du brome, ou un reste de formule $CH_2SiR^9R^{10}R^{11}$ ou un reste indanyle, formule dans laquelle

$R^9$, $R^{10}$ et $R^{11}$ sont identiques ou différents et représentent un reste alkyle linéaire ou ramifié ayant jusqu'à 3 atomes de carbone,

ou bien

$R^{13}$ et $R^{14}$ forment conjointement avec l'atome de carbone un noyau cyclopentyle, cyclohexyle ou cycloheptyle, ces noyaux pouvant être substitués, le cas échéant jusqu'à deux fois par un radical méthyle,

ou bien

$R^7$ et $R^8$ forment un reste de formule =O ou un reste à double liaison de formule

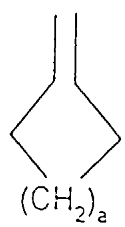

dans laquelle

a représente le nombre 2, 3, ou 4

et de leurs sels,
pour la préparation de médicaments destinés à abaisser le taux de lipoprotéines associées à l'ApoB-100.

**3.** Utilisation d'oxy-phényl-(phényl)-glycinolamides à substituant bicyclique suivant la revendication 1, dans lesquels

A représente le reste naphtyle ou un reste de formule

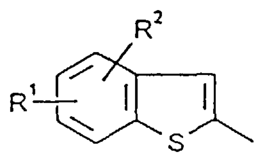 , 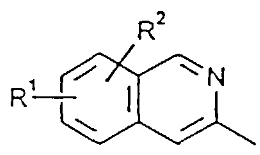 , 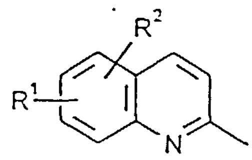 ,

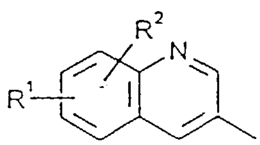 , 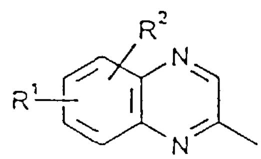 ou 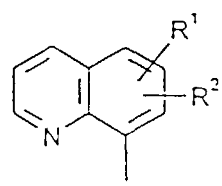

dans laquelle

$R^1$ et $R^2$ sont identiques ou différents et représentent l'hydrogène, le fluor, le chlore, le brome, un groupe hydroxy ou méthoxy,

D et E sont identiques ou différents et représentent l'hydrogène, un groupe cyclopropyle, cyclopentyle, cyclohexyle, azido, hydroxy, le fluor, le chlore, le brome, un groupe alkyle, alkoxy ou alcényle linéaire ou ramifié ayant dans chaque cas jusqu'à 5 atomes de carbone, un groupe pyrryle ou imidazolyle,
ou bien
un reste de formule $-NR^5SO_2-R^6$ dans laquelle

$R^5$ est l'hydrogène ou le groupe méthyle,
$R^6$ est un groupe alkyle linéaire ou ramifié ayant jusqu'à 3 atomes de carbone, benzyle ou phényle, qui est substitué le cas échéant par un radical méthyle ou éthyle,

L représente une liaison,
ou bien
un reste de formule

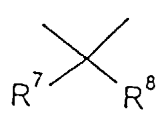

dans laquelle

$R^7$ est l'hydrogène, un groupe hydroxy, le fluor ou un groupe méthoxy,
$R^8$ est l'hydrogène, un groupe hydroxy, un groupe alcényle ou alkoxy linéaire ou ramifié ayant dans chaque cas jusqu'à 6 atomes de carbone, un groupe cyclopentyle, cyclohexyle, cycloheptyle, cyclo-octyle, cyclononyle, cyclo-décyle, cyclo-undécyle, cyclododécyle ou cyclohexényle, ou bien un groupe alkyle linéaire ou ramifié ayant jusqu'à 6 atomes de carbone, qui est substitué le cas échéant par un radical cyclopentyle, cyclohexyle, cycloheptyle, phényle ou tétrahydropyrannyle, ces radicaux pouvant eux-mêmes être substitués par du fluor, du chlore ou du brome, ou bien un reste de formule
$CH_2Si(R^9R^{10}R^{11})$ ou un reste indonyle,
formule dans laquelle
$R^9$, $R^{10}$ et $R^{11}$ représentent des groupes méthyle,

ou bien

$R^7$ et $R^8$ forment conjointement avec l'atome de carbone un noyau cyclopentyle, cyclohexyle ou cycloheptyle, ces noyaux étant éventuellement substitués jusqu'à deux fois par un radical méthyle, ou bien

$R^7$ et $R^8$ forment en commun un reste de formule =O ou un reste à double liaison de formule

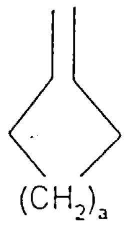

dans laquelle

a représente le nombre 2, 3 ou 4

et de leurs sels,
pour la préparation de médicaments destinés à abaisser le taux de lipoprotéines associées à l'ApoB-100.

**4.** Utilisation des oxy-phényl-(phényl)-glycinolamides à substituant bicyclique abaissant le taux de lipoprotéines associées à l'ApoB-100 suivant l'une des revendications 1 à 3 pour la préparation de médicaments destinés à abaisser le taux de VLDL dans le plasma.

**5.** Utilisation des oxy-phényl-(phényl)-glycinolamides à substituant bicyclique abaissant le taux de lipoprotéines associées à l'ApoB-100 suivant l'une des revendications 1 à 4 pour la préparation de médicaments destinés à abaisser le taux de triglycérides dans le plasma.

**6.** Utilisation des oxy-phényl-(phényl)-glycinolamides à substituant bicyclique abaissant le taux de lipoprotéines associées à l'ApoB-100 suivant l'une des revendications 1 à 5 pour la préparation de médicaments destinés à abaisser le taux de cholestérol dans le plasma.

**7.** Utilisation des oxy-phényl-(phényl) -glycinolamides à substituant bicyclique abaissant le taux de lipoprotéines associées à l'ApoB-100 d'après l'une des revendications 1 à 6, selon laquelle il s'agit de médicaments pour le traitement de maladies associées à des taux élevés dans le plasma de VLDL, d'ApoB-100, de LDL, de triglycérides ou de cholestérol.

**8.** Utilisation des oxy-phényl-(phényl)-glycinolamides à substituant bicyclique abaissant le taux de lipoprotéines associées à l'ApoB-100 suivant l'une des revendications 1 à 3 pour la préparation de médicaments destinés au traitement de l'adiposité.